# EUROPEAN PATENT SPECIFICATION

(11) **EP 4 512 379 B1**
(45) Date of publication and mention of the grant of the patent: **11.03.2026**
(21) Application number: 23799106.2
(22) Date of filing: 23.02.2023
(51) Int. Cl.: A61F 9/007

(54) **DELIVERY DEVICE FOR EYE IMPLANT**
FREISETZUNGSVORRICHTUNG FÜR AUGENIMPLANTAT
DISPOSITIF DE POSE POUR IMPLANT OCULAIRE

(30) Priority: 06.05.2022 CN 202210488561
(43) Date of publication of application: 26.02.2025
(73) Proprietor: Health Guard (Suzhou) Biomed. Technology Co., Ltd, Suzhou, Jiangsu 215123 (CN)
(72) Inventor: LIU, Hua, Suzhou, Jiangsu 215123 (CN); MA, Man, Suzhou, Jiangsu 215123 (CN)
(74) Representative: Barker Brettell LLP
(86) International application number: PCT/CN2023/077784
(87) International publication number: WO 2023/213116

(56) References cited:
- CN-A- 105 899 170
- CN-A- 113 208 806
- CN-A- 114 848 289
- CN-A- 114 848 290
- CN-U- 212 214 004
- CN-U- 213 910 449
- US-A1- 2004 054 374
- US-A1- 2010 191 224
- US-A1- 2014 213 958
- US-A1- 2015 133 946
- US-A1- 2016 158 049
- US-A1- 2017 156 848
- US-A1- 2020 390 601

## Description

### TECHNICAL FIELD

The present application relates to the technical field of medical devices, and in particular to an ocular implant delivery device.

### BACKGROUND

Glaucoma is a group of diseases that are commonly characterized by optic nerve atrophy and depression, visual field loss and decreased vision. Glaucoma is associated with increased intraocular pressure, usually due to the inability of the ocular drainage channels to adequately remove aqueous humor from the anterior chamber of the eye, or due to excessive aqueous humor production caused by the ciliary body in the eye. Patients often experience symptoms such as nausea and pain, if not treated in time, it will lead to vision loss. For the treatment of glaucoma, surgical filtration methods can be used to reduce intraocular pressure by creating a fluid flow path between the anterior chamber and the low-pressure area. Ocular implants can be positioned in the eye to drain fluid from the anterior chamber to multiple locations, such as the sub-Tenon's space, subconjunctival space, suprascleral vein, suprachoroidal space, Schlemm canal, and intrascleral space. Ocular implants are generally often prepared from a selection of animal-derived/non-animal-derived polymers, metals, and other materials, and are generally prevalent in tubular or tubelike form. According to the different mechanisms of reducing intraocular pressure in glaucoma, drainage of aqueous humor through the subconjunctival space and the superior scleral vein is more effective. The common surgical pathways are to penetrate the atrial angle area through the anterior chamber from a micro-corneal incision, reach the subconjunctival space through the sclera, or reach the suprachoroidal space through the anterior chamber angle. This type of operation requires a corresponding delivery mechanism to deliver the implant to the corresponding part of the eye.

CN105899170B discloses an inserter for treating glaucoma for positioning an intraocular shunt within an eye for treating glaucoma, which is used to position an intraocular shunt in the eye to treat glaucoma. The drive assembly of the inserter is a cylindrical member coupled to a needle via a needle recess and to a plunger via a plunger recess. The drive assembly further includes a sliding recess that longitudinally overlaps the needle recess and plunger recess. The sliding groove is used to couple the sliding assembly to the drive assembly, and the cylindrical member is driven to rotate in the longitudinal direction by movement of the sliding assembly on the housing to move the needle and the plunger in the axial direction. The cylindrical member is provided with three sections of grooves, and the structural design is relatively complex. Firstly, because the rotational fit of the grooves of the cylindrical member in the longitudinal direction is too long, which makes the structure insufficiently compact and leads to low accuracy; secondly, the cylindrical member cannot be assembled in one piece in a process, and the cylindrical member needs to be processed in two halves and then spliced together, which further affects the pushing accuracy; thirdly, the friction surface of the structure is larger when moving, which results in a larger dissipation of the pushing force and a low pushing efficiency; thus, it is necessary to make improvements to the ocular implant delivery device in the existing art.

US2020/390601 A1 discloses a system for preparation of an implant and ab interno insertion of the implant into an eye that comprises an actuator with a rack and pinion linkage. US2016/158049 A1 discloses a system for delivering a stent from a catheter sheath in a longitudinal direction.

### SUMMARY

The present application is defined by the appended claims. The purpose of the present application is to solve at least some of the problems of the ocular implant delivery device in the existing art, including low precision and difficulties in being molded in one piece, thereby affecting the pushing precision when splicing as well as having a large friction surface when pushing, resulting in a large dissipation of pushing force and low pushing efficiency. Therefore, there is provided an ocular implant delivery device, which is capable of realizing the precise delivery of ocular implant to the subconjunctival cavity, thereby reducing the intraocular pressure value of the patient with high intraocular pressure, realizing the simple structural design, and having better doctor experience.

Embodiments of the present application provide an ocular implant delivery device including: a housing, a rotating wheel, a push needle linkage assembly, a retracting linkage assembly, a sleeve, and a puncture needle. A through hole is provided at a distal end of the housing, and the sleeve extends from an inside of the housing to an outside of the housing through the through hole and is fixedly connected to the housing. The puncture needle runs through the sleeve, and an end of the puncture needle facing away from a needle tip of the puncture needle is fixedly connected to the retracting linkage assembly. A push needle of the push needle linkage assembly extends through an inner hole of the puncture needle, the rotating wheel is connected to an inside of the housing via rotating shafts, and an opening corresponding to the rotating wheel is provided on the housing. The push needle linkage assembly and the retracting linkage assembly are movably connected to end surfaces on two sides of the rotating wheel respectively, so that the push needle linkage assembly is driven to move in a direction toward the distal end of the housing or the retracting linkage assembly is driven to move in a direction facing away from the distal end of the housing by rotating wheel rotating.

The push needle linkage assembly and the retracting linkage assembly are respectively movably connected to the end surfaces of two sides of the rotating wheel through a groove-link shaft structure, so that by rotating the rotating wheel, a link shaft of the groove-link shaft structure slides along a movement trajectory as defined by a groove of the groove-link shaft structure to drive the push needle linkage assembly to move in the direction toward the distal end of the housing or to drive the retracting linkage assembly to move in a direction facing away from the distal end of the housing.

In some embodiments, the groove comprises two groove sections, one of which is an arc-shaped groove section, and the other of which is a variable radius groove section. An end of the variable radius groove section is communicated with an end of the arc-shaped groove section, and a distance between the center of the variable radius groove section and the center of the arc-shaped groove section gradually decreases from a connecting portion between the variable radius groove section and the arc-shaped groove section toward the other end of the variable radius groove section. With this ingenious groove structure design, the setting of the activity trajectory of the link shaft is realized, the structure is highly practical and reliable, and the implant can be delivered to the eye with as few working parts as possible.

In some embodiments, the groove further comprises a transition section arranged between the arc-shaped groove section and the variable radius groove section, two ends of the transition section is communicated with the arc-shaped groove section and the variable-radius groove section respectively. With providing the transition section, the user can feel obvious thrust feedback when the link shaft moves from the variable radius groove section to the transition section, thereby having a clear perception of the surgical process, which facilitates the surgical procedure.

In some embodiments, the push needle linkage assembly comprises a push needle, a push needle link, and a connecting seat, a proximal end of the push needle being fixedly connected to a distal end of the push needle link, and a proximal end of the push needle link being fixedly connected to the connecting seat. One of the link shaft and the groove is provided on a side surface of the connecting seat opposite to the rotating wheel, and the other of the groove and the link shaft is provided on the corresponding end surface of the rotating wheel. With this arrangement, the push needle link can be movably connected to the rotating wheel, and the implant can be transported as the rotating wheel rotates, and the moving distance of the implant can be accurately controlled, thereby improving the accuracy of the operation.

In some embodiments, the push needle linkage assembly further comprises a folded corner portion disposed at an end of the push needle link close to the connecting seat and is fixedly connected to the connecting seat, the folded corner portion having an extending direction deviating from an extending direction of the push needle link and extending from the push needle link in a direction facing away from the rotating wheel. With setting the folded corner portion, the distance between the push needle link and the retracting linkage assembly is reduced, the connecting seat can be connected to the side surface of the rotating wheel, and the push needle link can be disposed at the distal end of the rotating wheel, thereby making the internal structure of the ocular implant delivery device more compact.

In some embodiments, the retracting linkage assembly comprises a connecting head, a retracting link, and a connecting portion, the connecting head being fixedly connected to a distal end of the retracting link, and the connecting portion being fixedly connected to a proximal end of the retracting link. One of the link shaft and the groove is provided on a side surface of the connecting portion opposite to the rotating wheel, and the other of the groove and the link shaft is provided on the corresponding end surface of the rotating wheel. With this arrangement, the retracting link assembly can be connected to the rotating wheel, and the puncture needle can be retracted as the rotating wheel rotates, so as to realize the needle retraction operation after the implant is pushed, ensure the needle retraction process is linear, and improve the safety of the operation.

In some embodiments, the retracting linkage assembly further comprises a folded corner portion disposed at one end of the retracting link close to the connecting portion and fixedly connected to the connecting portion, and the connecting portion has an extending direction deviating from the retracting link and extends from the retracting link in a direction facing away from the rotating wheel. With setting the folded corner portion, the distance between the push needle link and the retracting link assembly is reduced, the connecting portion can be connected to the side of the rotating wheel, and the retracting link can be arranged at the distal end of the rotating wheel, thereby making the internal structure of the ocular implant delivery device more compact.

In some embodiments, the ocular implant delivery device further comprising a stop seat configured to limit a distance between the push needle linkage assembly and the retracting linkage assembly. With setting the stop seat to limit the distance between the push needle linkage assembly and the retracting linkage assembly, they form an integral structure, thereby improving space utilization.

In some embodiments, the stop seat comprises a first extending section and a second extending section intersecting each other. The first extending section is fixedly connected to the second extending section and is fixedly connected to the retracting linkage assembly or the push needle linkage assembly, and extends from the retracting linkage assembly to the push needle linkage assembly by a predetermined distance. The second extending section extends from the first extending section in a direction facing away from the first extending section, and the orthographic projection of the second extending section falls within the range of the retracting linkage assembly and the push needle linkage assembly along an extending direction of the first extending section. With this arrangement, the push needle linkage assembly or the retracting linkage assembly passes through the stop seat, so that the distance between the pushing needle linkage assembly and the retracting linkage assembly is limited to form an integral structure.

In some embodiments, a proximal end of the sleeve is fixedly connected to a sleeve seat which is disposed in the housing at a position close to the through hole, and the sleeve extends through the through hole on the housing to the outside of the housing. This arrangement can make the connection between the sleeve and the housing more secure, and facilitate the puncture needle to pass through the sleeve and puncture the position where the ocular implant needs to be delivered.

In some embodiments, a puncture needle seat is provided at a proximal end of the puncture needle and is fixedly connected to a distal end of the retracting linkage assembly, and the puncture needle is fixedly connected to the puncture needle seat and extends through the sleeve. With this arrangement, the puncture needle can be fixedly connected to the retracting linkage assembly, so that after the implantation of the ocular implant is completed, the puncture needle shrinks together with the retracting linkage assembly to achieve a straight needle retraction, thereby improving the safety of the operation.

In some embodiments, a first groove and an eccentric second link shaft are provided on two side end surfaces of the rotating wheel respectively, a third link shaft corresponding to the first groove is provided at a proximal end of the push needle linkage assembly, and a fourth groove corresponding to the second link shaft is provided at a proximal end of the retracting linkage assembly. The first groove comprises a variable radius groove section and an arc-shaped groove section, and the third link shaft slides from an end of the variable radius groove section to an end of the arc-shaped groove section of the first groove with the rotating wheel rotating toward the distal end. The fourth groove comprises an arc-shaped groove section and a variable radius groove section, and the second link shaft slides from an end of the arc-shaped groove section to an end of the variable radius groove section of the fourth groove with the rotating wheel rotating toward the distal end.

In some embodiments, a second groove and an eccentric first link shaft are provided on two side end surfaces of the rotating wheel respectively, a third groove corresponding to the first link shaft is provided at a proximal end of the push needle linkage assembly, and a fourth link shaft corresponding to the second groove is provided at a proximal end of the retracting linkage assembly. The third groove comprises a variable radius groove section and an arc-shaped groove section, and the first link shaft slides from an end of the variable radius groove section to an end of the arc-shaped groove section of the third groove with the rotating wheel rotating toward the distal end. The second groove comprises an arc-shaped groove section and a variable radius groove section, and the fourth link shaft slides from an end of the arc-shaped groove section to an end of the variable radius groove section of the second groove with the rotating wheel rotating toward the distal end.

In some embodiments, a first groove and a second groove are provided on end surfaces on two sides of the rotating wheel respectively, and a third link shaft corresponding to the first groove and a fourth link shaft corresponding to the second groove are provided on the push needle linkage assembly and the retracting linkage assembly respectively. The first groove comprises a variable radius groove section and an arc-shaped groove section, and the third link shaft slides from an end of the variable radius groove section to an end of the arc-shaped groove section of the first groove with the rotating wheel rotating toward the distal direction. The second groove comprises an arc-shaped groove section and a variable radius groove section, and the fourth link shaft slides from an end of the arc-shaped groove section to an end of the variable radius groove section of the second groove with the rotating wheel rotating toward the distal end.

In some embodiments, an eccentric first link shaft and an eccentric second link shaft are respectively provided end surfaces on two sides of the rotating wheel. A third groove corresponding to the first link shaft is provided at a proximal portion of the push needle linkage assembly and comprises a variable radius groove section and an arc-shaped groove section, and the first link shaft slides from an end of the variable radius groove section to an end of the arc-shaped groove section of the third groove with the rotating wheel rotating toward the distal end. A fourth groove is provided at a proximal end of the retracting linkage assembly and comprises an arc-shaped groove section and a variable radius groove section, and the second link shaft slides from an end of the arc-shaped groove section to an end of the variable radius groove section of the fourth groove with the rotating wheel rotating toward the distal end.

In some embodiments, the housing comprises an upper housing and a lower housing fixedly connected to the upper housing, the upper housing and the lower housing each comprise a cylinder portion, a gripping portion, and a conical portion that are fixedly connected together; a rotating wheel mount is disposed on an inner wall surface of the cylinder portion of the housing, and the rotating wheel is fitted on the rotating wheel mount via the rotating shafts; a stop block is provided on an inner wall surface of at least one of the cylinder portion, the gripping portion, and the conical portion, and the stop block is configured to limit the push needle linkage assembly and the retracting linkage assembly in a radial direction. With providing the gripping portion, it is convenient for the doctor to hold the ocular implant delivery device for surgery, and by providing the rotating wheel mount, it is convenient to fit the rotating wheel and enable the rotating wheel to rotate flexibly. By providing the stop block in the housing, the push needle link and the retracting link can be further radially limited to prevent radial displacement, thereby improving the accuracy of the surgery.

In some embodiments, the rotating wheel is provided with teeth on an outer circumferential surface, and the housing is provided with an operating-sliding sleeve at a position corresponding to the opening, the operating-sliding sleeve being slidably fitted with the housing; and the operating-sliding sleeve is provided with a rack portion on an inner wall, the rack portion intermeshing with the teeth on the rotating wheel. By providing the operating-sliding sleeve, it is convenient for the doctor to push the operating-sliding sleeve by hand to perform surgery. Compared with the scheme of directly rotating the rotating wheel to perform surgery, the flexibility and convenience of the operation of the ocular implant delivery device can be greatly improved, making the surgical process more labor-saving and more efficient.

In some embodiments, the housing is provided with a spring sheet on an outer wall, the spring sheet extending to the outside of the housing and cooperating with the rack portion on an inner wall of the operating-sliding sleeve. With providing the spring sheet, the doctor will have obvious tactile feedback when pushing the operating sleeve to perform surgery. Every time the rotating wheel moves a tooth, the spring sheet will intermesh with the tooth, which can effectively prevent the rotating wheel from rotating in the opposite direction due to misoperation during surgery, thereby improving the safety of the operation of the ocular implant delivery device. In addition, with providing the spring sheet, the doctor can avoid using too much force during surgery and the rotating wheel rotating too fast, which will affect the quality of the surgery, further improving the safety of the surgery.

In some embodiments, the push needle linkage assembly and the retracting linkage assembly are each provided with a positioning part, which is provided with a positioning hole, and the positioning holes are opposite to each other; and the housing is provided with a positioning through hole at a position corresponding to the positioning holes, and a positioning rod is provided in the positioning through hole. With this arrangement, the retracting linkage assembly and the push needle linkage assembly inside the ocular implant delivery device can be protected so that they will not produce relative displacement before the ocular implant surgery, thereby ensuring the accuracy of the moving distance of the ocular implant during the surgery, ensuring the accuracy of the surgery, and improving the quality of the surgery.

It should be noted that the distal end refers to the end facing away from the doctor during an ocular implant operation, and the proximal end refers to the end close to the doctor during ocular implant operation. It can be interpreted that the distal end refers to the end close to the patient's eye that needs operation, and the proximal end is the end facing away from the patient's eye.

The embodiments of the present application has at least some of the following positive effects:
1) In embodiments of the present application, the ocular implant delivery device including a push needle linkage assembly, a retracting linkage assembly and a groove-link shaft structure ingeniously realizes the delivery of the implant and the retraction of the puncture needle through the one-way rotation of the rotating wheel, which can greatly reduce the volume of the ocular implant delivery device, and can make the push needle move in a straight line during the operation, improve the quality of the operation, and can retract the puncture needle in a straight line after the delivery of the ocular implant is completed, thereby improving the safety of the operation.
2) In embodiments of the present application, the ocular implant delivery device has a simple structure and small volume, and is easy to operate, which can improve the efficiency of the doctor's operation and the safety of the operation.
3) The ocular implant delivery device in embodiments of the present application, the rotating wheel is connected to the housing via the rotating shaft, the gap between the rotating wheel and the housing is small, and the structural design is more compact. In the embodiments of the present application, the rotation of the rotating wheel can be realized that the implantation operation of the ocular implant can be realized, the friction is small, the loss of the pushing force is reduced, the operation is more labor-saving, and the system stability and reliability are higher.

### BRIEF DESCRIPTION OF THE DRAWINGS

FIG. 1 is a structural schematic view of an ocular implant delivery device according to the Embodiment 1 of the present application;
FIG. 2 is a schematic structural view of a splitting structure of a delivery device according to the Embodiment 1 of the present application (a direction of an arrow is a rotation direction of the rotating wheel);
FIG. 3 is a structural schematic view of a groove on a side of the rotating wheel opposite to a retracting link according to the Embodiment 1 of the present application (a direction of an arrow is a rotation direction of the rotating wheel);
FIG. 4 is a structural schematic view of a groove on a side of the rotating wheel opposite to a push needle link according to the Embodiment 1 of the present application (a direction of an arrow is a direction of an activity trajectory of a third link shaft in a first groove);
FIG. 5 is a structural schematic view of a push needle link according to the Embodiment 1 of the present application;
FIG. 6 is a structural schematic view of a retracting link according to the Embodiment 1 of the present application (a direction of an arrow is a direction of an activity trajectory of a second link shaft in a fourth groove);
FIG. 7 is a structural schematic view of a lower housing according to the Embodiment 1 of the present application;
FIG. 8 is a schematic cross-sectional view of an inner structure of housing;
FIG. 9 is a structural schematic view of an assembly structure of a sleeve, a puncture needle, an ocular implant, and a push needle in FIG. 8;
FIG. 10 is a structural schematic view of a second link shaft on a rotating wheel in a fourth groove on a retracting link in an initial state according to the Embodiment 1 of the present application (a direction of an arrow is a direction of an activity trajectory of a second link shaft in a fourth groove);
FIG. 11 is a structural schematic view of a state of a second link shaft on a rotating wheel sliding to an arc-shaped groove section in a fourth groove on a retracting link according to the Embodiment 1 of the present application (a direction of an arrow is a direction of an activity trajectory of a second link shaft in a fourth groove);
FIG. 12 is a structural schematic view of a state of a second link shaft on a rotating wheel sliding to an variable radius groove section in a fourth groove on a retracting link according to the Embodiment 1 of the present application (a direction of an arrow is a direction of an activity trajectory of a second link shaft in a fourth groove);
FIG. 13 is a schematic structural view of a splitting structure of a delivery device according to the Embodiment 2 of the present application (a direction of an arrow is a rotation direction of the rotating wheel);
FIG. 14 is a schematic structural view of a rotating wheel where a side surface facing a retracting link according to the Embodiment 2 of the present application (a direction of an arrow is a direction of an activity trajectory of a fourth link shaft in a second groove);
FIG. 15 is a schematic structural view of a rotating wheel where a side surface facing a push needle link according to the Embodiment 2 of the present application (a direction of an arrow is a direction of an activity trajectory of a fourth link shaft in a second groove);
FIG. 16 is a structural schematic view of an assembly structure of a rotating wheel, a retracting link, and a push needle link according to the Embodiment 3 of the present application (a direction of an arrow is a rotation direction of the rotating wheel);
FIG. 17 is a structural schematic view of a retracting link according to the Embodiment 3 of the present application (a direction of an arrow is a direction of an activity trajectory of a second link shaft in a fourth groove);
FIG. 18 is a structural schematic view of a push needle link according to Embodiment 3 of the present application (a direction of an arrow is a direction of an activity trajectory of a first link shaft in a third groove);
FIG. 19 is a structural schematic view of an ocular implant according to Embodiment 4 of the present application;
FIG. 20 is a structural schematic view of an assembly structure of an operating-sliding sleeve, a rotating wheel, and a spring sheet of an ocular implant delivery device according to Embodiment 4 of the present application.

The reference signs in the drawings are as follows:
1. Housing; 11. Cylinder portion; 12. Gripping portion; 13. Conical portion; 14. Through hole; 15. Opening; 16. Stop block; 17. Rotating wheel mount; 2. Sleeve; 3. Puncture needle; 31. Puncture needle seat; 4: Rotating wheel; 41. First groove; 411. Arc-shaped groove section; 412. Variable radius groove section; 413. Transition section; 42. Second groove; 421. Arc-shaped groove section; 422. Variable radius groove section; 423. Transition section; 43. First link shaft; 44. Second link shaft; 45. Rotating shaft; 46. Teeth; 5. Push needle linkage assembly; 51. Push needle; 52. Push needle link; 521. Folded corner portion; 53. Connecting seat; 54. Third link shaft; 55. Third groove; 551. Arc-shaped groove section; 552. Variable radius groove section; 553. Transition section; 56. Positioning hole; 6. Retracting linkage assembly; 61. Connector; 62. Retracting link; 621.Folded corner portion; 63. Connecting portion; 63. Connecting portion; 64. Stop seat; 65. Fourth link shaft; 66. Fourth groove; 661. Arc-shaped groove section; 662. Variable radius groove section; 663. Transition section; 67. Positioning hole; 7. Operating-sliding sleeve; 71. Rack portion; 72. Spring sheet; 8. Implant; 81. Plug; 9. Positioning rod.

### DETAILED DESCRIPTION

The technical scheme of the present application is described clearly and completely below through embodiments. Obviously, the described embodiments are only a part of the embodiments of the present application, rather than all the embodiments. Based on the embodiments in the present application, all other embodiments obtained by ordinary technicians in the field without creative work are within the scope of protection of the present application.

It should be noted that the distal end refers to the end facing away from the doctor during ocular implant operation, and the proximal end refers to the end close to the doctor during an ocular implant operation. It can be interpreted that the distal end refers to the end close to the patient's eye that needs operation, and the proximal end is the end facing away from the patient's eye.

### Embodiment 1

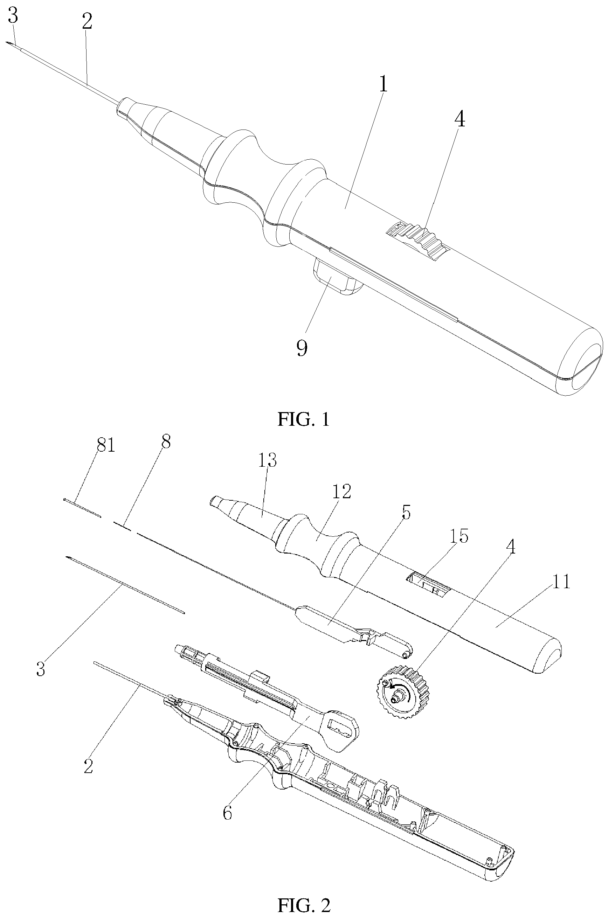

A ocular implant delivery device as shown in FIGS. 1-12, which includes a housing 1, a rotating wheel 4, a push needle linkage assembly 5, a retracting linkage assembly 6, a sleeve 2, and puncture needle 3. The push needle linkage assembly 5 and the retracting linkage assembly 6 are respectively movably connected to the end surfaces of two sides of the rotating wheel 4 through a groove-link shaft structure. A link shaft of the groove-link shaft structure can slide in a groove of the groove-link shaft structure serving as an activity trajectory, and the push needle linkage assembly is driven to move in the direction toward the distal end of the housing or the retracting linkage assembly is driven to move in a direction facing away from the distal end of the housing by rotating wheel rotating.

The housing 1 is a split molded part, and the structure of the housing 1 can be selected in many ways; for example, as shown in FIG. 2, the housing 1 is divided into an upper housing and a lower housing from the proximal end to the distal end, and the upper housing and the lower housing can be fixedly connected by glue, by buckles, or by bolts. Another solution is that the housing is divided into two parts along the longitudinal direction, and the two parts are connected by threaded connection, buckle connection, glue fixing connection, or the like.

The structure shown in FIG. 1 is taken as an example for explanation below. The housing 1 includes an upper housing and a lower housing, which are fixedly connected. The upper housing and the lower housing each includes a cylindrical portion 11, a gripping portion 12 and a conical portion 13 from the proximal end to the distal end. The cylindrical portion 11, the gripping portion 12 and the conical portion 13 are fixedly connected or integrally formed. A through hole 14 for connecting the sleeve 2 is disposed on the conical portion 13. With the gripping portion 12, it is convenient for the doctor to hold the ocular implant delivery device during the operation, and the specific shape of the housing 1 can be adjusted and designed according to the convenience of operation.

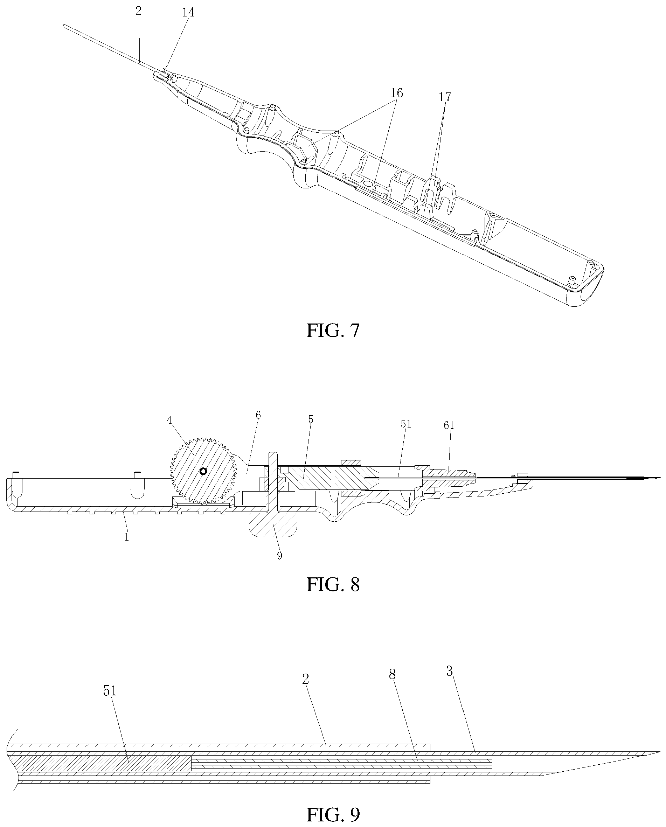

As shown in FIG. 7, a rotating wheel mount 17 is disposed on the inner wall surface of the cylindrical portion of the housing 1, and the rotating wheel 4 is assembled on the rotating wheel mount 17 via a rotating shaft. The rotating wheel mount 17 is provided to facilitate the assembly of the rotating wheel 4 and enable the rotating wheel 4 to rotate flexibly.

The housing 1 is provided with an opening 15 at a position corresponding to the rotating wheel 4, so that the doctor or other surgical operator can operate the surgical procedure by rotating the rotating wheel at the opening 15. Alternatively, the outer periphery of the rotating wheel 4 can be partially protruded from the opening or be flush with the opening of the housing 1.

Inside the housing 1, a stop block 16 is disposed on the inner wall surface of at least one of the cylindrical portion 11, the gripping portion 12 and the conical portion 13, and the stop block 16 is configured to limit the radial position of the push needle linkage assembly 5 and the retracting linkage assembly 6. With sufficient limit on the housing 1, the retracting link 62 and the push needle link 52 can be prevented from being deviated radially in the housing 1, thereby improving the accuracy of the operation.

As shown in FIG. 8 and FIG. 9, a sleeve 2 is disposed at the distal end of the housing 1. The sleeve 2 is a hollow tubular object, which is fixedly connected to the distal through hole 14 of the housing 1 by gluing or injection molding or other clamping mechanisms. The sleeve 2 is used to protect the needle tube of the puncture needle 3 and to support the trabecular meshwork tissue when the puncture needle 3 enters the subconjunctival cavity during the operation, thereby limiting the insertion length of the puncture needle.

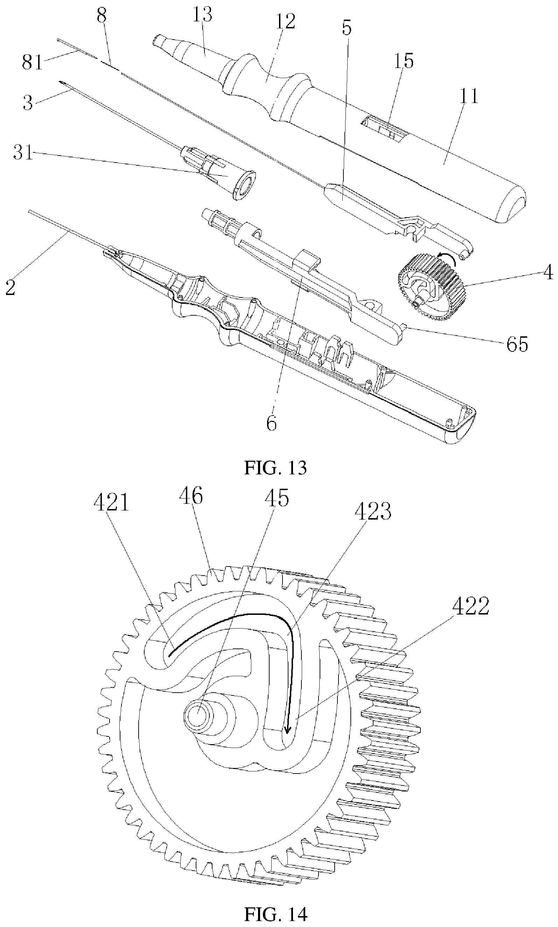

Optionally, a sleeve seat is provided at the proximal end of the sleeve 2, and the sleeve 2 is fixedly connected to the sleeve seat by gluing or injection molding. The sleeve seat is disposed in the housing 1 at a position close to the through hole 14, and the sleeve 2 is fixedly connected to the housing 1 with the connection method that can be a fixed connection method such as gluing, injection molding or clamping. The puncture needle 3 is a needle with a hollow cylindrical structure, and its material can be a material specified in the medical field. An oblique opening is provided at the needle tip of the puncture needle 3. The puncture needle 3 passes through the hole of the sleeve 2, and has a clearance fit with the inner wall of the sleeve 2 to enable the puncture needle 3 to pass freely in the sleeve 2. The puncture needle 3 can be directly fixedly connected to the distal end of the retracting linkage assembly 6, or the puncture needle 3 can be fixedly connected to the puncture needle seat 31 by gluing or injection molding, or other clamping mechanisms. As shown in FIG. 13, the puncture needle seat 31 is fixedly connected to the retracting linkage assembly 6.

The puncture needle 3 can pass through the hollow hole of the sleeve 2 and extend to the outer side of the distal end of the sleeve 2. The oblique opening of the puncture needle 3 completely exposes a length of the sleeve 2, thereby controlling the depth of the puncture needle 3 penetrating into the conjunctiva of the eye. The puncture needle 3 and the puncture needle seat 31 are retained in the internal space composed of the upper housing and lower housing, and they can be retracted only and cannot move toward the distal end.

The implant 8 can be a tubular object made of a flexible material, and the tubular inner cavity of the implant 8 forms an aqueous humor outlet channel. The implant 8 has a certain arch along the axial direction. Before the operation, the implant 8 is placed in the inner cavity of the puncture needle 3 and is retained by the distal end of the push needle. A certain friction force is generated between the outer wall of the implant 8 and the inner wall of the puncture needle 3, which can stabilize the position of the implant 8 in the hole of the puncture needle 3.

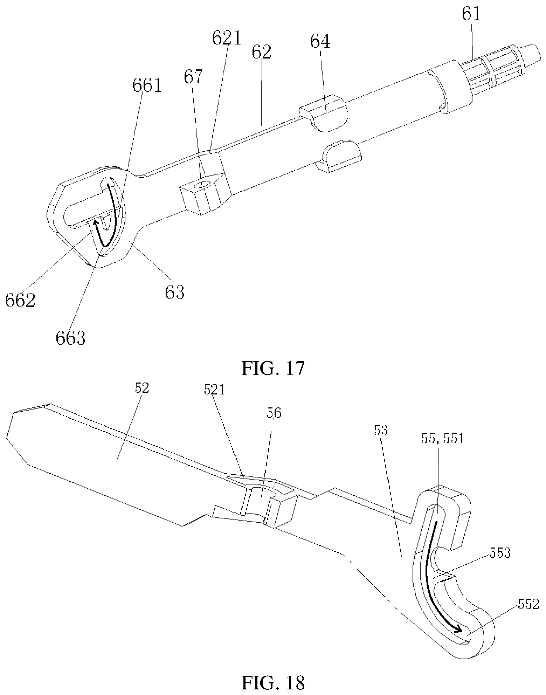

The push needle linkage assembly 5 includes a push needle 51, a push needle link 52, and a connecting seat 53. The proximal end of the push needle 51 is fixedly connected to the distal end of the push needle link 52. The proximal end of the push needle link 52 is fixedly connected to the connecting seat 53. A third link shaft 54 or a third groove 55 is provided on the side surface of the connecting seat 53 opposite to the rotating wheel 4. As shown in FIG. 18, the push needle link is movably connected to the rotating wheel 4 through a groove link shaft mechanism. In order to reduce the distance between the push needle link 52 and the retracting link 62 and make the internal structure of the ocular implant delivery device more compact, a folded corner portion 521 is provided at the connection portion between the push needle link 52 and the connecting seat 53. The extending direction of the folded corner portion deviates from the extending direction of the push needle link and extends from the push needle link to the direction facing away from the rotating wheel. By setting the folded corner portion, the connecting seat 53 can be connected to the side of the rotating wheel 4, and the push needle link 52 can be set at the distal end of the rotating wheel 4.

The push needle 51 is connected to the distal end of the push needle link by gluing or injection molding, or other clamping mechanisms. The distal end of the push needle 51 passes through the hollow hole of the puncture needle 3. As the push needle linkage assembly 5 moves, the push needle 51 can move toward the distal end of the puncture needle 3 and push the implant 8 installed in the puncture needle 3 into the puncture site of the patient's eye. As the push needle 51 moves, the implant 8 is pushed to the patient's intraocular subconjunctival cavity. The tubular inner cavity of the implant 8 forms an aqueous humor outflow channel to discharge the aqueous humor in the eye, thereby achieving the purpose of treatment.

The retracting linkage assembly 6 includes a connector 61, a retracting link 62 and a connecting portion 63. The connector 61 is fixedly connected to the distal end of the retracting link 62, and the connecting portion 63 is fixedly connected to the proximal end of the retracting link 62. A fourth link shaft 65 or a fourth groove 66 is provided on a side surface of the connecting portion 63 opposite to the rotating wheel 4. The retracting link 62 is movably connected to the rotating wheel 4 through a groove link shaft mechanism. In order to reduce the distance between the push needle link 52 and the retracting link 62, and make the internal structure of the ocular implant delivery device more compact, a folded corner portion 621 is provided at the connection portion between the retracting link 62 and the connecting portion 63. The extending direction of the connecting portion deviates from the retracting link, and extends from the retracting link to the direction facing away from the rotating wheel. By setting the folded corner portion, the connecting portion 63 can be connected to the side of the rotating wheel 4, and the retracting link 62 can be set at the distal end of the rotating wheel. A stop seat 64 is provided on the retracting link, and the push needle link 52 passes through the stop seat 64, so that the two form an integral structure. It can be understood that in other application scenarios, a stop seat can also be provided on the push needle link 52, so that after the retracting link passes through the stop seat on the push needle link, it forms an integral structure with the push needle link. The design of this integral structure makes the space utilization high and the internal structure more compact. Optionally, the stop seat 64 may include a first extending section and a second extending section intersecting each other. The first extending section is fixedly connected to the second extending section and the retracting link 62 or the push needle link 52, and extends a preset distance from the retracting link 62 to the push needle link 52. The second extending section extends from the first extending section to the direction facing away from the first extending section and the positive projection of the second extending section along the extending direction of the first extending section falls within the range of the retracting link 62 and the push needle link 52. With this arrangement, the push needle link 52 or the retracting link 62 passes through the stop seat, so that the distance between the push needle link and the retracting link is limited to form an integral structure.

Optionally, the push needle link 52 and the retracting link 62 are each provided with a positioning part, and the positioning part on the push needle link 52 is provided with a positioning hole 56, and the positioning part on the positioning part on is provided with a positioning hole 67 opposite to the positioning hole 56, and a positioning through hole is provided on the housing 1 at a position corresponding to the positioning holes, and a positioning rod 9 is provided in the positioning through hole. With this arrangement, the retracting linkage assembly 6 and the push needle linkage assembly 5 inside the ocular implant delivery device can be protected, so that they will not produce relative displacement before the ocular implant operation, the moving distance accuracy of the ocular implant during the operation is guaranteed, the accuracy of the operation is ensured, and the quality of the operation is improved.

In the above scheme, the connection mode of the push needle linkage assembly 5 the rotating wheel 4 and the connection mode of the retracting linkage assembly 6 and the rotating wheel 4 can be that the groove is disposed on the side of the rotating wheel and the link shaft is disposed on the push needle link 52 and the retracting link 62; the groove is disposed at the proximal end of the push needle link 52 or the retracting link 62, and eccentric link shafts are respectively disposed on the two sides of the rotating wheel 4; the groove is disposed on one side of the rotating wheel 4 and the link shaft is disposed on the other side of the rotating wheel 4, and one of the proximal end of the push needle link 52 and the proximal end of the retracting link 62 is provided with a link shaft, the other of them is provided with a groove. Therefore, there may be a plurality of permutations and combinations of implementation schemes.

As a first embodiment, a third link shaft 54 is provided at the proximal end of the push needle link 52, a fourth groove 66 is provided at the proximal end of the retracting link 62, and a first groove 41 is provided the rotating wheel 4 on the end face opposite to the push needle link 52, and an eccentric second link shaft 44 is provided on the rotating wheel 4 on the end face of opposite to the retracting link 62.

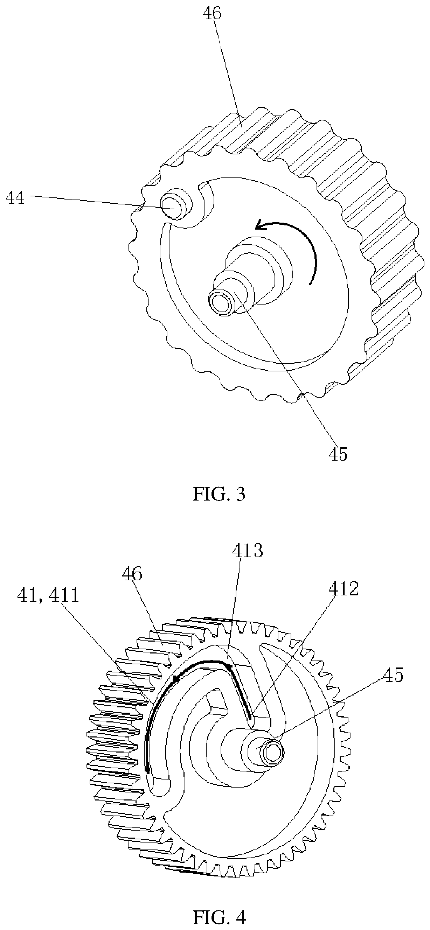

As shown in FIG. 4, the first groove 41 provided on the rotating wheel 4 includes a variable radius groove section 412 and an arc-shaped groove section 411. The center of the arc-shaped groove section 411 coincides with the center of the rotating wheel 4. The two groove sections communicate. The variable radius groove section 412 can be selected from groove sections of various shapes, for example, an involute groove section. When the rotating wheel 4 rotates toward the distal end, as shown in the direction of the arrow in FIG. 4, the third link shaft 54 on the push needle link 52 slides from the end of the variable radius groove section 412 toward the end of the arc-shaped groove section 411; when in the variable radius groove section 412, the distance between the third link shaft 54 and the rotating shaft 45 of the rotating wheel 4 gradually increases; then entering the arc-shaped groove section 41, the distance between the link shaft 54 and the rotating shaft 45 of the rotating wheel 4 remains unchanged.

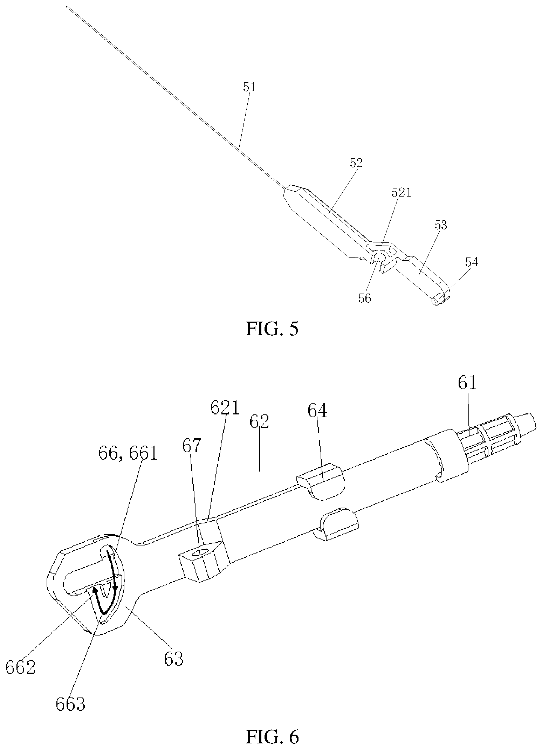

As shown in FIG. 6, the fourth groove 66 arranged at the proximal end of the retracting link 62 includes an arc-shaped groove section 661 and a variable radius groove section 662 that communicate each other. The variable radius groove section 662 can be a groove section of various shapes, for example, an arc-shaped groove of an approximate involute shape. When the rotating wheel 4 rotates toward the distal end, as shown in the arrow direction of FIG. 6, the second link shaft 44 on the rotating wheel 4 slides from the end of the arc-shaped groove section 661 of the fourth groove 66 toward the end of the variable radius groove section 662; when in the circular arc-shaped groove section 661, the distance between the center of the circle of the second link shaft 44 and the arc-shaped groove section 661 remains unchanged; then entering the variable radius groove section 662, the distance between the second link shaft 44 and the center of the arc-shaped groove section 661 gradually decreases.

When the ocular implant delivery device of this embodiment is used, the delivery operation of the ocular implant and the needle retracting operation of the puncture needle can be completed by rotating the rotating wheel 4, thereby completing the ocular implant operation with high precision.

The specific process is as follows: in the initial state, the third link shaft 54 on the push needle linkage assembly 5 is disposed at the end of the variable radius groove section 412 of the first groove 41 on the corresponding end surface of the rotating wheel 4, and the eccentric second link shaft 44 on the end surface on the other side of the rotating wheel 4 is disposed at the end of the arc-shaped groove section 661 of the fourth groove 66 at the distal end of the retracting link 62. Moreover, the positioning hole 56 of the positioning part of the push needle linkage assembly 5 and the positioning hole 67 of the positioning part of the retracting linkage assembly 6 are just opposite to each other and face the positioning through hole on the housing 1. The positioning rod 9 passes through the positioning through hole on the housing, and the positioning hole 56 on the push needle linkage assembly 5, and the positioning hole 67 on the retracting linkage assembly 6 in sequence from the outside to the inside, so that the ocular implant delivery device is in a locked state.

The implant 8 is pre-installed in the inner hole of the puncture needle 3 to abut against the push needle 51 of the push needle linkage assembly 5, and the distal end of the implant 8 is limited by a plug 81 to prevent the implant 8 from falling. Before implantation, the positioning rod 9 and the plug 81 are pulled out to unlock the ocular implant delivery device, and then the puncture needle 3 is used to puncture the surgical site of the patient. Because the length of the puncture needle 3 exposing out of the sleeve 2 is fixed, the sleeve 2 can limit the puncture depth of the puncture needle 3. After the puncture is completed, the implantation operation begins.

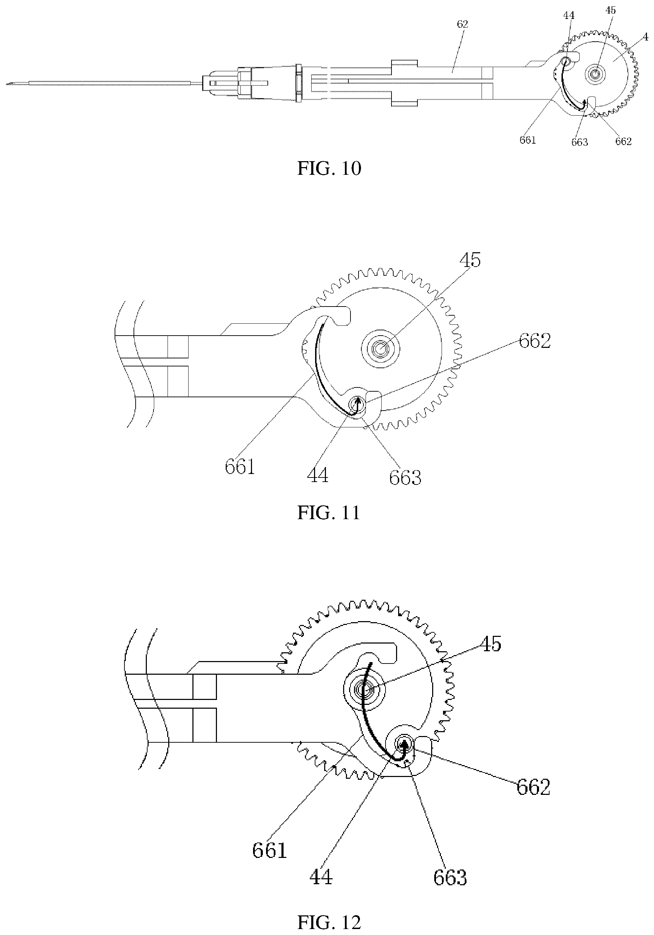

In the first stage, as shown in the direction of the arrow in FIG. 2, the rotating wheel 4 is rotated toward the distal end. As shown in the direction of the arrow in FIG. 4, the third link shaft 54 on the push needle link 52 slides from the end of the variable radius groove section 412 of the first groove 41 on the rotating wheel 4 toward the arc-shaped groove section 411. During this process, the distance between the third link shaft 54 on the push needle link 52 and the rotating shaft 45 of the rotating wheel 4 gradually increases, and the push needle link 52 is radially limited in the housing 1, so that the push needle link 52 can only move toward the distal end of the housing 1, driving the push needle 51 to move toward the distal end, and pushing the implant 8 through the puncture hole to reach the patient's intraocular subconjunctival cavity; at the same time, the direction of the arrow as shown in FIGS. 10-12, the second link shaft 44 on the other side of the rotating wheel 4 slides in the arc-shaped groove section 661 of the fourth groove 66 at the proximal end of the retracting link 62. Since the contact arc radius remains unchanged, there is no axial force between the second link shaft 44 and the fourth groove 66, and therefore the retracting linkage assembly 6 will not move in the axial direction.

When the rotating wheel 4 continues to be rotated in the same direction to enter the second stage, the third link shaft 54 on the push needle link 52 enters the arc-shaped groove section 411 of the first groove 41. Because the distance between the arc-shaped groove section 411 and the center of the rotating wheel 4 remains unchanged, the push needle link 52 no longer moves when the rotating wheel 4 is rotated; at the same time, as shown in FIGS. 11-12, the second link shaft 44 on the rotating wheel 4 enters the variable radius groove section 662 of the fourth groove 66 on the retracting link 62. Because when the second link shaft 44 moves from the arc-shaped groove section 661 to the end of the variable radius groove section 662, the distance between the second link shaft 44 and the center of the arc-shaped groove section 661 is constantly decreasing, thereby driving the retracting link 62 to move toward the proximal direction. A stop block 16 is provided in the housing 1 to limit the radial movement of the retracting link 62, so that it can only move along the axial direction of the housing 1 toward the proximal direction, driving the puncture needle 3 to move into the sleeve 2 to complete the needle retraction operation.

At this point, the process of delivering the implant 8 to a specific position in the eye is completed. For operators such as doctors, they only need to operate the rotating wheel 4 to complete the implant delivery process, which is simple and easy.

Optionally, in the first groove 41 on the rotating wheel 4 corresponding to the push needle linkage assembly 5, a transition section 413 is provided between the variable radius groove section 412 and the arc-shaped groove section 411 and can be an arc-shaped transition section. In the fourth groove 66 provided at the proximal end of the retracting link 62, a transition section 663 is provided between the arc-shaped groove section 661 and the variable radius groove section 662 and can be an arc-shaped transition section. With providing the transition section 413 between the variable radius groove section 412 and the arc-shaped groove section 411 of the first groove 41, obvious thrust feedback will appear, and the user will feel that the implant pushing has ended and can have a clear perception of the surgical process, when transitioning from the aforementioned first stage to the second stage, after the link shaft 54 on the push needle link 52 ends the movement in the variable radius groove section 412 and enters the transition section 413, which is convenient for the subsequent needle retracting operation. Similarly, with providing a transition section 663 between the arc-shaped groove section 661 and the variable radius groove section 662 in the groove at the proximal end of the retracting link 62, when transitioning from the aforementioned first stage to the second stage, after the link shaft 45 on the rotating wheel 4 ends the movement of the arc-shaped groove section 661 on the retracting link 62 and enters the transition section 663, there will be obvious thrust feedback, and the user will feel that the implant pushing has been completed. According to the status of the operation, the user can decide whether to retract the puncture needle 3 into the sleeve 2 by retracting the linkage assembly 6 to end the ocular implant operation.

As another variation of this embodiment, a link shaft 54 can be provided on the end surface of the rotating wheel 4 opposite to the push needle link 52, and a groove can be provided on the end surface opposite to the retracting link 62. Correspondingly, a groove can be provided on the push needle link 52, and a link shaft 65 can be provided on the retracting link 62. The working principle of this scheme is similar to the working principle described above, and the technical effect achieved is also the same, which will not be described in detail here.

### Embodiment 2

As shown in FIGS. 13-15, as a second embodiment, a first groove 41 and a second groove 42 are respectively provided on both side surfaces of the rotating wheel 4, and the push needle linkage assembly 5 is provided with a third link shaft 54 corresponding to the groove, and the retracting linkage assembly 6 is provided with a fourth link shaft 65 corresponding to the groove.

The first groove on the rotating wheel 4 corresponding to the third link shaft 54 on the push needle linkage assembly 5 includes a variable radius groove section 412 and an arc-shaped groove 411, and the center of the arc-shaped groove section 411 coincides with the center of the rotating wheel 4. The variable radius groove section 412 is communicated with the arc-shaped groove section 411, and when the rotating wheel 4 rotates toward the distal end, the third link shaft 54 on the push needle link 52 slides from the end of the variable radius groove section 412 of the first groove 41 toward the end of the arc-shaped groove section 411. In the variable radius groove section 412, the distance between the third link shaft 54 and the rotating shaft 45 of the rotating wheel 4 gradually increases, and then the third link shaft 54 entering the arc-shaped groove section 411, the distance between the third link shaft 54 and the rotating shaft 45 of the rotating wheel 4 remains unchanged.

The second groove on the rotating wheel 4 corresponding to the fourth link shaft 65 of the retracting linkage assembly 6 includes an arc-shaped groove section 421 and a variable radius groove section 422. The shape of the variable radius groove section 422 can be selected from various options, for example, an arc-shaped groove with an approximately involute shape. The arc-shaped groove section 421 and the variable radius groove section 422 communicate, when the rotating wheel 4 rotates toward the distal end, the fourth link shaft 65 on the retracting linkage assembly 6 slides from the end of the arc-shaped groove section 421 of the second groove 42 toward the end of the variable radius groove section 422. In the arc-shaped groove section 421, the distance between the fourth link shaft 65 and the rotating shaft 45 of the rotating wheel 4 remains unchanged, and then the fourth link shaft 65 entering the variable radius groove section 422, the distance between the fourth link shaft 65 and the rotating shaft 45 of the rotating wheel 4 gradually decreases.

As shown in FIG. 13, the ocular implant delivery device of this embodiment can complete the delivery operation of the ocular implant and the needle retracting operation of the puncture needle by rotating the rotating wheel 4 when in use, and can complete the ocular implant operation with high precision. The specific process is that in the initial state, the third link shaft 54 on the push needle link 52 is disposed at the end of the variable radius groove section 412 of the first groove 41 on the corresponding end surface of the rotating wheel 4, and the fourth link shaft 65 on the retracting link 62 is disposed at the end of the arc-shaped groove section 421 of the second groove 42 on the corresponding end surface of the rotating wheel 4. Moreover, the positioning hole 56 of the positioning parts of the push needle linkage assembly 5 and the positioning hole 67 of the positioning part of the retracting linkage assembly 6 are just opposite to each other and face the positioning through hole on the housing 1. The positioning rod 9 passes through the positioning through hole on the housing, and the positioning hole 56 on the push needle linkage assembly and the positioning hole 67 on the retracting linkage assembly from the outside to the inside, so that the ocular implant delivery device is in a locked state.

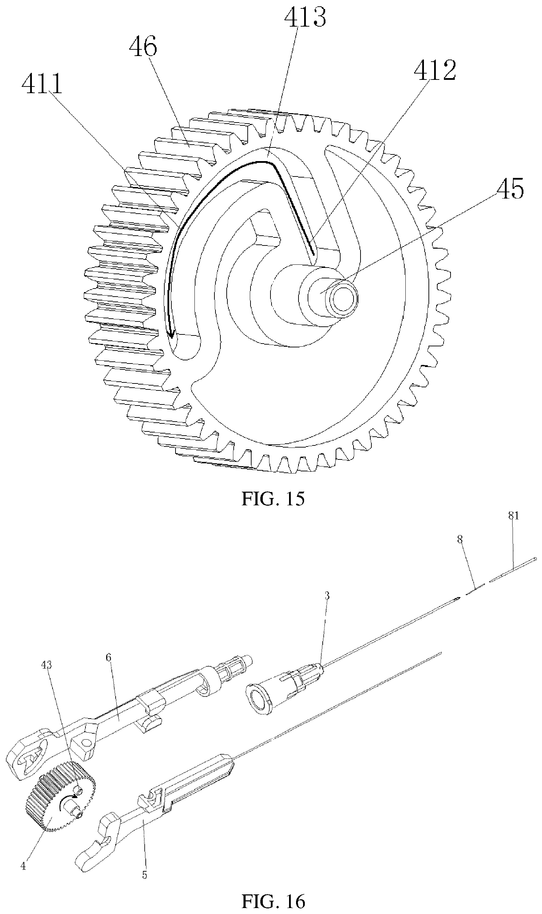

The implant 8 is pre-installed into the inner hole of the puncture needle 3 to abut against the push needle 51 of the push needle linkage assembly 5. A plug 81 is provided at the distal end of the implant 8 to limit the position and prevent the implant 81 from falling. Before implantation, the positioning rod 9 and the plug 81 are pulled out to unlock the ocular implant delivery device, and then the puncture needle 3 is used to puncture the surgical site of the patient. Because the length of the puncture needle 3 exposing out of the sleeve 2 is fixed, the sleeve 2 can limit the puncture depth of the puncture needle 3. After the puncture is completed, the rotating wheel 4 is rotated toward the distal direction. When the rotating wheel 4 rotates, in the first stage, as shown in the direction of the arrow in FIG. 15, the third link shaft 54 on the push needle linkage assembly 5 slides from the end of the variable radius groove section 412 of the first groove 41 toward the arc-shaped groove section 411. During this process, the distance between the third link shaft 54 on the push needle link 52 and the center of the rotating wheel 4 gradually increases, and the push needle link 52 is radially limited in the housing 1, so that the push needle link 52 can only move toward the distal end of the housing 1, driving the push needle link 51 to move toward the distal end of the housing, and pushing the ocular implant through the puncture hole 3 to reach the patient's intraocular subconjunctival cavity. At the same time, as shown in the direction of the arrow in FIG. 14, the fourth link shaft 65 on the retracting link 62 slides in the arc-shaped groove section 421 of the second groove 42 on the other side end surface of the rotating wheel 4, and the distance between the fourth link shaft 65 and the center of the rotating wheel 4 remains unchanged, so the retracting link 62 will not move.

With continuing to rotate the rotating wheel 4 in the same direction to enter the second stage, the third link shaft 45 on the push needle link 52 enters the arc-shaped groove section 411 of the first groove 41 on the rotating wheel 4. Since the distance between the arc-shaped groove section 411 and the center of the rotating wheel 4 remains unchanged, and the push needle link 52 no longer moves when the rotating wheel 4 is rotated. At the same time, the fourth link shaft 65 on the retracting link 62 enters the variable radius groove section 422 of the second groove 42, the distance between the fourth link shaft 65 and the rotating shaft 45 of the rotating wheel 4 is constantly decreasing when the fourth link shaft 65 moves from the arc-shaped groove section 421 to the end of the variable radius groove section 422, and thus the retracting link 62 can be driven to move toward the proximal direction. A stop block 16 is provided in the housing 1, and thus the radial movement of the retracting link 62 can be limited, so that it can only move along the axial direction of the housing 1 toward the proximal direction, driving the puncture needle 3 to move into the sleeve 2, and completing the needle retracting operation.

At this point, the process of delivering the implant 8 to a specific position in the eye is completed.

Optionally, in the first groove 41 on the rotating wheel 4 corresponding to the push needle linkage assembly 5, a transition section 413 is provided between the variable radius groove section 412 and the arc-shaped groove section 411, and the transition section 413 can be an arc-shaped transition section. In the second groove 42 on the rotating wheel 4 corresponding to the retracting linkage assembly 6, a transition section 423 is provided between the arc-shaped groove section 421 and the variable radius groove section 422, and the transition section 423 can be an arc-shaped transition section. By providing a transition section between the variable radius groove section 412 and the arc-shaped groove section 411 of the first groove 41, obvious thrust feedback will appear, and the user will feel that the implant pushing has ended, and can have a clear perception of the surgical process, when transitioning from the aforementioned first stage to the second stage, after the third link shaft 54 on the push needle link 52 ends the movement of the variable radius groove section 412 and enters the transition section, which is convenient for the subsequent needle retracting operation. Similarly, by providing a transition section 423 between the arc-shaped groove section 421 and the variable radius groove section 422 of the second groove 42, obvious thrust feedback will appear, and the user will feel that the pushing of the implant has been completed, when transitioning from the aforementioned first stage to the second stage, after the fourth link shaft 65 on the retracting link 62 completes the movement of the arc-shaped groove section 421 and enters the transition section 423. According to the status of the operation, the user can decide whether to retract the puncture needle 3 into the sleeve 2 by retracting the linkage assembly 6 to complete the ocular implant operation.

### Embodiment 3

As shown in FIGS. 16-18, as a third implementation scheme, different from the above embodiments, an eccentric first link shaft 43 and an eccentric second link shaft 44 are respectively provided on both side surfaces of the rotating wheel 4, and a third groove 55 and a fourth groove 66 are respectively provided at the proximal end of the push needle link 52 and the proximal end of the retracting link 62.

The third groove 55 disposed at the proximal end of the push needle link 52 includes a variable radius groove section 552 and an arc-shaped groove 551. The variable radius groove section 552 and the arc-shaped groove section 551 communicate. When the rotating wheel 4 rotates toward the distal end, the first link shaft 43 on the rotating wheel 4 slides from the end of the variable radius groove section 552 toward the end of the arc-shaped groove section 551. In the variable radius groove section 552, the distance between the first link shaft 43 and the center of the arc-shaped groove section 551 gradually increases, and then the first link shaft 43 entering the arc-shaped groove section 551, the distance between the first link shaft 43 and the center of the arc-shaped groove section 551 remains unchanged.

The fourth groove 66 disposed at the proximal end of the retracting link 62 includes an arc-shaped groove section 661 and a variable radius groove section 662 communicating each other. The variable radius groove section 662 can be selected from groove sections of various shapes, for example, an arc-shaped groove of an approximately involute shape. When the rotating wheel 4 rotates toward the distal end, the second link shaft 44 on the rotating wheel 4 slides from the end of the arc-shaped groove section 661 of the fourth groove 66 toward the end of the variable radius groove section 662. In the arc-shaped groove section 661, the distance between the second link shaft 44 and the center of the arc-shaped groove section 661 remains unchanged, and then entering the variable radius groove section 662, the distance between the second link shaft 44 and the center of the arc-shaped groove section 661 gradually decreases.

The ocular implant delivery device of this embodiment as shown in FIG. 16 can complete the delivery operation of the ocular implant and the needle retracting operation of the puncture needle by rotating the rotating wheel 4 when in use, and can complete the ocular implant operation with high precision. The specific process is that in the initial state, the third link shaft 54 on the push needle link 52 is disposed at the end of the variable radius groove section 412 of the first groove 41 on the corresponding end surface of the rotating wheel 4, and the fourth link shaft 65 on the retracting link 62 is disposed at the end of the arc-shaped groove section 421 of the second groove 42 on the corresponding end surface of the rotating wheel. Moreover, the positioning hole 56 of the positioning part of the push needle linkage assembly 5 and the positioning hole 67 of the positioning part of the retracting linkage assembly 6 are just opposite to each other, and are directly opposite to the positioning through hole on the housing 1, and the positioning rod 9 passes through the positioning through hole on the housing, the positioning hole 56 on the push needle link 52, and the positioning hole 67 on the retracting link 62 from the outside to the inside, so that the ocular implant delivery device is in a locked state.

The implant 8 is pre-installed in the inner hole of the puncture needle 3 to abut against the push needle 51 of the push needle linkage assembly 5, and the distal end of the implant 8 is limited by a plug 81 to prevent the implant 8 from falling. Before implantation, the positioning rod 9 and the plug 81 are pulled out to unlock the ocular implant delivery device, and then the puncture needle 3 is used to puncture the surgical site of the patient. Since the length of the puncture needle exposing out of the sleeve 2 is fixed, the sleeve 2 can limit the puncture depth of the puncture needle 3. After the puncture is completed, the rotating wheel 4 is rotated in a direction towards the distal direction. When the rotating wheel 4 rotates, in the first stage, as shown in the arrow direction of FIG. 18, the first link shaft 43 on the rotating wheel 4 corresponding to the push needle link 52 slides from the end of the variable radius groove section 552 of the third groove 55 at the proximal end of the push needle link 52 toward the arc-shaped groove section 551. In this process, the distance between the first link shaft 43 on the rotating wheel 4 and the center of the arc-shaped groove section 551 of the third groove 55 gradually increases, and the push needle link 52 is radially limited in the housing 1, so that the push needle link 52 can only move toward the distal end of the housing 1, driving the push needle 51 to move toward the distal end, and pushing the ocular implant through the puncture hole 3 to reach the patient's intraocular subconjunctival cavity. At the same time, as shown in the arrow direction of FIG. 17, the second link shaft 44 on the other side of the rotating wheel 4 slides in the arc-shaped groove section 661 of the fourth groove 66 at the proximal end of the retracting link 62, so the retracting link does not move.

With continuing to rotate the rotating wheel 4 in the same direction to enter the second stage until the first link shaft 43 on the rotating wheel 4 enters the arc-shaped groove section 551 of the third groove 55 at the proximal end of the push needle link 52, since the radius of the arc-shaped groove section 551 remains unchanged, the push needle link 52 no longer moves when the rotating wheel is rotated; at the same time, the second link shaft 44 on the other side of the rotating wheel 4 enters the variable radius groove section 662 of the fourth groove 66 at the proximal end of the retracting link 62. The distance between the second link shaft 44 and the center of the arc-shaped groove section 661 is constantly decreasing when the second link shaft 44 moves from the arc-shaped groove section 661 of the fourth groove 66 to the end of the variable radius groove section 662, thereby driving the retracting link 62 to move toward the proximal direction. A stop block 16 is provided in the housing 1 to limit the radial movement of the retracting link 62 so that it can only move along the axial direction of the housing 1 toward the proximal direction, driving the puncture needle 3 to move into the sleeve 2 to complete the needle retracting operation.

At this point, the process of delivering the implant 8 to a specific position in the eye is completed.

Optionally, in the third groove 55 at the proximal end of the push needle link 52, a transition section 553 is provided between the variable radius groove section 552 and the arc-shaped groove section 551 and can be an arc-shaped transition section. In the fourth groove 66 at the proximal end of the retracting link 62, a transition section 663 is provided between the arc-shaped groove section 661 and the variable radius groove section 662 and can be an arc-shaped transition section. By providing the transition section 553 between the variable radius groove section 552 and the arc-shaped groove section 551 in the third groove 55 at the proximal end of the push needle link 52, obvious thrust feedback will appear, and the user will feel that the implant pushing has ended and can have a clear perception of the surgical process, when transitioning from the aforementioned first stage to the second stage, after the first link shaft 43 on the rotating wheel 4 ends the movement of the variable radius groove section 552 and enters the transition section 553, which is convenient for the subsequent needle retracting operation. Similarly, by setting a transition section 663 between the arc-shaped groove section 661 and the variable radius groove section 662 in the fourth groove 66 at the proximal end of the retracting link 62, obvious thrust feedback will appear, and the user will feel that the pushing of the implant 8 has been completed, when transitioning from the aforementioned first stage to the second stage, after the second link shaft 44 on the rotating wheel 4 completes the movement of the arc-shaped groove section 661 and enters the transition section 663. According to the status of the operation, the user can decide whether to retract the puncture needle 3 into the sleeve 2 by retracting the linkage assembly 6 to end the ocular implant operation.

### Embodiment 4

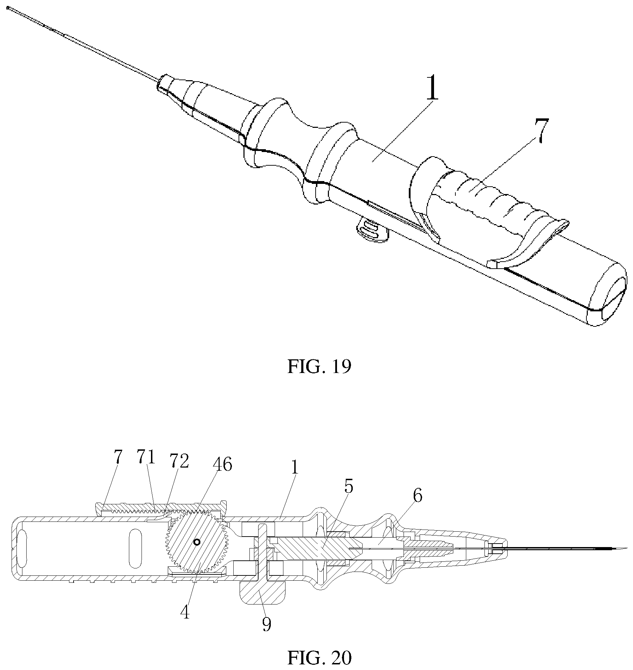

As shown in FIG. 19 and 20, in order to improve convenience, teeth 46 can be set on the outer peripheral surface of the rotating wheel 4. For example, a gear is used as the rotating wheel. An operating-sliding sleeve 7 is further provided at a position corresponding to the opening 15 on the housing 1, and a rack portion 71 intermeshing with the teeth on the rotating wheel 4 is provided on the inner wall of the operating-sliding sleeve 7. The operating-sliding sleeve is slidably fitted with the housing, and the specific settings can be various. As shown in FIGS. 19 and 20, for example, a slide groove can be disposed on the side wall of the housing, and the operating-sliding sleeve is slidably fitted with the slide groove, or the sleeve can be set in an annular shape and sleeved on the housing. By setting the operating-sliding sleeve 7, it is convenient for the doctor to push the operating-sliding sleeve 7 by hand to perform operation. Compared with the solution of directly rotating the rotating wheel 4 to perform operation, the flexibility and convenience of the operation of the ocular implant delivery device can be greatly improved, making the surgical process more labor-saving and more efficient.

Optionally, a spring sheet 72 is provided on the outer wall of the cylindrical portion of the housing 1, and the spring sheet 72 extends to the outside of the housing 1 and cooperates with the rack portion 71 on the inner wall of the operating-sliding sleeve 7. By providing the spring sheet 72, when the doctor pushes the operating-sliding sleeve 7 to perform operation, each time the rotating wheel 4 moves a tooth, the spring sheet 72 will be engaged with the tooth 46, which can effectively prevent the rotating wheel 4 from rotating in the opposite direction due to misoperation during operation, thereby improving the safety of the operation of the ocular implant delivery device. In addition, With providing the spring sheet 72, it can be prevented that the doctor uses too much force during operation, and the rotating wheel 4 rotates too fast, which affects the quality of the operation, thereby further improving the safety of the operation.

When the ocular implant delivery device of the present application is in use, the implant 8 that meets the surgical conditions is first loaded into the sterile puncture needle 3, and then the patient's eyes are treated accordingly according to the surgical conditions, such as anesthesia, sterilization, or the like. When the operation is to be performed, the positioning rod 9 on the housing 1 is pulled out, the lock of the push needle link 52 and the retracting link 62 is released, the puncture needle 3 of the ocular implant delivery device is used to puncture the part of the patient where the ocular implant needs to be implanted, and the puncture depth of the puncture needle 3 can be limited by the sleeve 2 to improve the accuracy of the operation. After the puncture is in place, the doctor holds the gripping portion of the ocular implant delivery device and slowly pushes the rotating wheel 4 or pushes the operating-sliding sleeve 7 toward the distal end of the housing 1. During this process, the third link shaft 54 on the push needle link 52 moves toward the distal end of the housing 1 under the action of the rotating wheel 4. Since a stop block 16 is provided in the housing 1 to limit the push needle link 52, the radial displacement of the push needle link 52 can be avoided, so that it can only move linearly toward the distal end of the housing 1, driving the push needle 51 to move toward the puncture needle 3, and pushing the ocular implant through the puncture hole 3 to reach the patient's intraocular subconjunctival cavity. With continuing to rotate the wheel 4 in the same direction or pushing the operating-sliding sleeve 7 toward the distal end, the retracting link 62 moves toward the proximal direction. When moving, the retracting link 62 is affected by the stop block 16 in the housing 1, it can only move linearly from the distal end to the proximal end of the housing 1, driving the puncture needle 3 to retract into the sleeve 2, completing the needle retracting operation. The ocular implant delivery device is used to deliver the implant 8 to a specific position in the eye, and the tubular inner cavity of the implant 8 forms an aqueous humor outflow channel to discharge the aqueous humor in the eye, thereby achieving the purpose of reducing intraocular pressure.

The ocular implant delivery device of the present application has a compact structure, ingenious design, and is easy to operate, which can greatly improve the accuracy, safety, and efficiency of operation, reduce the difficulty and risk of surgical operations, alleviate the pain of patients, and improve the success rate of operation.

## Claims

1. An ocular implant delivery device, comprising a housing (1), a rotating wheel (4), a push needle linkage assembly (5), a retracting linkage assembly (6), a sleeve (2), and a puncture needle (3),
wherein a through hole (14) is provided at a distal end of the housing (1), and the sleeve (2) extends from an inside of the housing (1) to an outside of the housing (1) through the through hole (14) and is fixedly connected to the housing (1);
wherein the puncture needle (3) runs through the sleeve (2), and an end of the puncture needle (3) facing away from a needle tip of the puncture needle (3) is fixedly connected to the retracting linkage assembly (6);
wherein a push needle (51) of the push needle linkage assembly (5) extends through an inner hole of the puncture needle (3), the rotating wheel (4) is connected to an inside of the housing (1) via rotating shafts (45), and an opening (15) corresponding to the rotating wheel (4) is provided on the housing (1); **characterized in that**
the push needle linkage assembly (5) and the retracting linkage assembly (6) are movably connected to end surfaces on two sides of the rotating wheel (4) respectively, so that the push needle linkage assembly (5) is driven to move in a direction toward the distal end of the housing (1) or the retracting linkage assembly (6) is driven to move in a direction facing away from the distal end of the housing (1) by the rotating wheel (4) rotating;
and wherein:
the push needle linkage assembly (5) and the retracting linkage assembly (6) are respectively movably connected to the end surfaces of two sides of the rotating wheel (4) through a groove-link shaft structure, so that by rotating the rotating wheel (4), a link shaft of the groove-link shaft structure slides along a movement trajectory as defined by a groove of the groove-link shaft structure to drive the push needle linkage assembly (5) to move in the direction toward the distal end of the housing (1) or to drive the retracting linkage assembly (6) to move in a direction facing away from the distal end of the housing (1).

2. The ocular implant delivery device according to claim 1, wherein the groove comprises two groove sections, one of which is an arc-shaped groove section, and the other of which is a variable radius groove section; and
an end of the variable radius groove section is communicated with an end of the arc-shaped groove section, and a distance between a center of the variable radius groove section and a center of the arc-shaped groove section gradually decreases from a connecting portion between the variable radius groove section and the arc-shaped groove section toward the other end of the variable radius groove section.

3. The ocular implant delivery device according to claim 1, wherein the push needle linkage assembly (5) comprises the push needle (51), a push needle link (52), and a connecting seat (53), a proximal end of the push needle (51) being fixedly connected to a distal end of the push needle link (52), and a proximal end of the push needle link (52) being fixedly connected to the connecting seat (53); and
one of the link shaft and the groove is provided on a side surface of the connecting seat (53) opposite to the rotating wheel (4), and the other of the groove and the link shaft is provided on the corresponding end surface of the rotating wheel (4).

4. The ocular implant delivery device according to claim 1, wherein the retracting linkage assembly (6) comprises a connector (61), a retracting link (62), and a connecting portion (63), the connector (61) being fixedly connected to a distal end of the retracting link (62), and the connecting portion (63) being fixedly connected to a proximal end of the retracting link (62); and
one of the link shaft and the groove is provided on a side surface of the connecting portion (63) opposite to the rotating wheel (4), and the other of the groove and the link shaft is provided on the corresponding end surface of the rotating wheel (4).

5. The ocular implant delivery device according to claim 1, wherein a proximal end of the sleeve (2) is fixedly connected to a sleeve seat which is disposed in the housing (1) at a position close to the through hole (14), and the sleeve (2) extends through the through hole (14) on the housing (1) to the outside of the housing (1).

6. The ocular implant delivery device according to claim 1, wherein a puncture needle seat (31) is provided at a proximal end of the puncture needle (3) and is fixedly connected to a distal end of the retracting linkage assembly (6), and the puncture needle (3) is fixedly connected to the puncture needle seat (31) and extends through the sleeve (2).

7. The ocular implant delivery device according to claim 1, wherein a first groove (41) and an eccentric second link shaft (44) are provided on end surfaces on two sides of the rotating wheel (4) respectively, a third link shaft (54) corresponding to the first groove (41) is provided at a proximal end of the push needle linkage assembly (5), and a fourth groove corresponding to the second link shaft (44) is provided at a proximal end of the retracting linkage assembly (6);
the first groove (41) comprises a variable radius groove section (412) and an arc-shaped groove section (411), and the third link shaft (54) slides from an end of the variable radius groove section (412) to an end of the arc-shaped groove section (411) of the first groove (41) as the rotating wheel (4) rotates toward the distal end; and
the fourth groove (66) comprises an arc-shaped groove section (411) and a variable radius groove section (412), and the second link shaft (44) slides from an end of the arc-shaped groove section (411) to an end of the variable radius groove section (412) of the fourth groove (66) as the rotating wheel (4) rotates toward the distal end.

8. The ocular implant delivery device according to claim 1, wherein a second groove (42) and an eccentric first link shaft (43) are provided on end surfaces on two sides of the rotating wheel (4) respectively, a third groove (55) corresponding to the first link shaft (43) is provided at a proximal end of the push needle linkage assembly (5), and a fourth link shaft (65) corresponding to the second groove (42) is provided at a proximal end of the retracting linkage assembly (6);
the third groove (55) comprises a variable radius groove section (552) and an arc-shaped groove section (551), and the first link shaft (43) slides from an end of the variable radius groove section (552) to an end of the arc-shaped groove section (551) of the third groove (55) as the rotating wheel (4) rotating toward the distal end; and
the second groove (42) comprises an arc-shaped groove section (421) and a variable radius groove section (422), and the fourth link shaft (65) slides from an end of the arc-shaped groove section (421) to an end of the variable radius groove section (422) of the second groove (42) as the rotating wheel (4) rotating toward the distal end.

9. The ocular implant delivery device according to claim 1, wherein a first groove (41) and a second groove (42) are provided on two side surfaces of the rotating wheel (4) respectively, and a third link shaft (54) corresponding to the first groove (41) and a fourth link shaft (65) corresponding to the second groove (42) are provided on the push needle linkage assembly (5) and the retracting linkage assembly (6) respectively;
the first groove (41) comprises a variable radius groove section (412) and an arc-shaped groove section (411), and the third link shaft (54) slides from an end of the variable radius groove section (412) to an end of the arc-shaped groove section (411) of the first groove (41) as the rotating wheel (4) rotates toward the distal direction; and
the second groove (42) comprises an arc-shaped groove section (421) and a variable radius groove section (422), and the fourth link shaft (65) slides from an end of the arc-shaped groove section (421) to an end of the variable radius groove section (422) of the second groove (42) as the rotating wheel (4) rotates toward the distal end.

10. The ocular implant delivery device according to claim 1, wherein an eccentric first link shaft (43) and an eccentric second link shaft (44) are respectively provided on two side surfaces of the rotating wheel (4);
a third groove (55) corresponding to the first link shaft (43) is provided at a proximal portion of the push needle linkage assembly (5) and comprises a variable radius groove section (552) and an arc-shaped groove section (521), and the first link shaft (43) slides from an end of the variable radius groove section (552) to an end of the arc-shaped groove section (521) of the third groove (55) as the rotating wheel (4) rotates toward the distal end; and
a fourth groove (66) is provided at a proximal end of the retracting linkage assembly (6) and comprises an arc-shaped groove section (661) and a variable radius groove section (662), and the second link shaft (44) slides from an end of the arc-shaped groove section (661) to an end of the variable radius groove section (662) of the fourth groove (66) as the rotating wheel (4) rotates toward the distal end.

11. The ocular implant delivery device according to claim 1, wherein the housing (1) comprises an upper housing and a lower housing fixedly connected to the upper housing, the upper housing and the lower housing each comprise a cylinder portion (11), a gripping portion (12), and a conical portion (13) that are fixedly connected together;
a rotating wheel mount (17) is disposed on an inner wall surface of the cylinder portion (11) of the housing (1), and the rotating wheel (4) is fitted on the rotating wheel mount (17) via the rotating shafts (45); and
a stop block (16) is provided on an inner wall surface of at least one of the cylinder portion (11), the gripping portion (12), and the conical portion (13), and the stop block (16) is configured to limit the push needle linkage assembly (5) and the retracting linkage assembly (6) in a radial direction.

12. The ocular implant delivery device according to claim 1, wherein the rotating wheel (4) is provided with teeth (46) on an outer circumferential surface, and the housing (1) is provided with an operating-sliding sleeve (7) at a position corresponding to the opening (15), the operating-sliding sleeve being slidably fitted with the housing (1); and
the operating-sliding sleeve (7) is provided with a rack portion on an inner wall, the rack portion intermeshing with the teeth (46) on the rotating wheel (4).

13. The ocular implant delivery device according to claim 12, wherein the housing (1) is provided with a spring sheet (72) on an outer wall, the spring sheet (72) extending to the outside of the housing (1) and cooperating with the rack portion on an inner wall of the operating-sliding sleeve (7).

14. The ocular implant delivery device according to claim 1, wherein the push needle linkage assembly (5) and the retracting linkage assembly (6) are each provided with a positioning part, which is provided with a positioning hole, and the positioning holes are opposite to each other; and
the housing (1) is provided with a positioning through hole at a position corresponding to the positioning holes, and a positioning rod is provided in the positioning through hole.

## Patentansprüche

1. Vorrichtung zur Einbringung eines Augenimplantats, umfassend ein Gehäuse (1), ein Drehrad (4), eine Schubnadel-Pleuelanordnung (5), eine Rückzugs-Pleuelanordnung (6), eine Hülse (2) und eine Punktionsnadel (3),
wobei ein Durchgangsloch (14) an einem distalen Ende des Gehäuses (1) vorgesehen ist und sich die Hülse (2) von einer Innenseite des Gehäuses (1) zu einer Außenseite des Gehäuses (1) durch das Durchgangsloch (14) erstreckt und fest mit dem Gehäuse (1) verbunden ist;
wobei die Punktionsnadel (3) durch die Hülse (2) verläuft und ein von einer Nadelspitze der Punktionsnadel (3) abgewandtes Ende der Punktionsnadel (3) fest mit der Rückzugs-Pleuelanordnung (6) verbunden ist;
wobei sich eine Schubnadel (51) der Schubnadel-Pleuelanordnung (5) durch ein inneres Loch der Punktionsnadel (3) erstreckt, das Drehrad (4) über Drehwellen (45) mit einer Innenseite des Gehäuses (1) verbunden ist und eine dem Drehrad (4) entsprechende Öffnung (15) am Gehäuse (1) vorgesehen ist; **dadurch gekennzeichnet, dass**
die Schubnadel-Pleuelanordnung (5) und die Rückzugs-Pleuelanordnung (6) jeweils beweglich mit Endflächen auf zwei Seiten des Drehrades (4) verbunden sind, sodass durch die Drehung des Drehrades (4) die Schubnadel-Pleuelanordnung (5) in Richtung des distalen Endes des Gehäuses (1) bewegt wird oder die Rückzugs-Pleuelanordnung (6) in eine Richtung weg vom distalen Ende des Gehäuses (1) bewegt wird;
und wobei:
die Schubnadel-Pleuelanordnung (5) und die Rückzugs-Pleuelanordnung (6) jeweils über eine Nut-Pleuelwellenstruktur beweglich mit den Endflächen der beiden Seiten des Drehrades (4) verbunden sind, sodass durch Drehen des Drehrades (4) eine Pleuelwelle der Nut-Pleuelwellenstruktur entlang einer durch eine Nut der Nut-Pleuelwellenstruktur definierten Bewegungsbahn gleitet, um die Schubnadel-Pleuelanordnung (5) in Richtung des distalen Endes des Gehäuses (1) zu bewegen oder um die Rückzugs-Pleuelanordnung (6) in eine Richtung weg vom distalen Ende des Gehäuses (1) zu bewegen.

2. Vorrichtung zur Einbringung eines Augenimplantats nach Anspruch 1, wobei die Nut zwei Nutabschnitte umfasst, von denen einer ein bogenförmiger Nutabschnitt und der andere ein Nutabschnitt mit variablem Radius ist; und
ein Ende des Nutabschnitts mit variablem Radius mit einem Ende des bogenförmigen Nutabschnitts in Verbindung steht und ein Abstand zwischen einer Mitte des Nutabschnitts mit variablem Radius und einer Mitte des bogenförmigen Nutabschnitts von einem Verbindungsabschnitt zwischen dem Nutabschnitt mit variablem Radius und dem bogenförmigen Nutabschnitt zum anderen Ende des Nutabschnitts mit variablem Radius hin allmählich abnimmt.

3. Vorrichtung zur Einbringung eines Augenimplantats nach Anspruch 1, wobei die Schubnadel-Pleuelanordnung (5) die Schubnadel (51), einen Schubnadel-Pleuel (52) und einen Verbindungssitz (53) umfasst, wobei ein proximales Ende der Schubnadel (51) fest mit einem distalen Ende des Schubnadel-Pleuels (52) verbunden ist und ein proximales Ende des Schubnadel-Pleuels (52) fest mit dem Verbindungssitz (53) verbunden ist; und
eine der Pleuelwelle und der Nut an einer Seitenfläche des Verbindungssitzes (53) gegenüber dem Drehrad (4) vorgesehen ist, und die andere der Nut und der Pleuelwelle an der entsprechenden Endfläche des Drehrades (4) vorgesehen ist.

4. Vorrichtung zur Einbringung eines Augenimplantats nach Anspruch 1, wobei die Rückzugs-Pleuelanordnung (6) ein Verbindungselement (61), einen Rückzugspleuel (62) und einen Verbindungsabschnitt (63) umfasst, wobei das Verbindungselement (61) fest mit einem distalen Ende des Rückzugspleuels (62) verbunden ist und der Verbindungsabschnitt (63) fest mit einem proximalen Ende des Rückzugspleuels (62) verbunden ist; und
eine der Pleuelwelle und der Nut an einer Seitenfläche des Verbindungsabschnitts (63) gegenüber dem Drehrad (4) vorgesehen ist, und die andere der Nut und der Pleuelwelle an der entsprechenden Endfläche des Drehrads (4) vorgesehen ist.

5. Vorrichtung zur Einbringung eines Augenimplantats nach Anspruch 1, wobei ein proximales Ende der Hülse (2) fest mit einem Hülsensitz verbunden ist, der in dem Gehäuse (1) an einer Stelle nahe dem Durchgangsloch (14) angeordnet ist, und sich die Hülse (2) durch das Durchgangsloch (14) an dem Gehäuse (1) zur Außenseite des Gehäuses (1) erstreckt.

6. Vorrichtung zur Einbringung eines Augenimplantats nach Anspruch 1, wobei ein Punktionsnadelsitz (31) an einem proximalen Ende der Punktionsnadel (3) vorgesehen ist und fest mit einem distalen Ende der Rückzugs-Pleuelanordnung (6) verbunden ist, und die Punktionsnadel (3) fest mit dem Punktionsnadelsitz (31) verbunden ist und sich durch die Hülse (2) erstreckt.

7. Vorrichtung zur Einbringung eines Augenimplantats nach Anspruch 1, wobei eine erste Nut (41) und eine exzentrische zweite Pleuelwelle (44) jeweils an den Endflächen auf zwei Seiten des Drehrades (4) vorgesehen sind, eine dritte Pleuelwelle (54), die der ersten Nut (41) entspricht, an einem proximalen Ende der Schubnadel-Pleuelanordnung (5) vorgesehen ist und eine vierte Nut, die der zweiten Pleuelwelle (44) entspricht, an einem proximalen Ende der Rückzugs-Pleuelanordnung (6) vorgesehen ist;
die erste Nut (41) einen Nutabschnitt mit variablem Radius (412) und einen bogenförmigen Nutabschnitt (411) umfasst, und die dritte Pleuelwelle (54) von einem Ende des Nutabschnitts mit variablem Radius (412) zu einem Ende des bogenförmigen Nutabschnitts (411) der ersten Nut (41) gleitet, wenn sich das Drehrad (4) in Richtung des distalen Endes dreht; und
die vierte Nut (66) einen bogenförmigen Nutabschnitt (411) und einen Nutabschnitt mit variablem Radius (412) umfasst, und die zweite Pleuelwelle (44) von einem Ende des bogenförmigen Nutabschnitts (411) zu einem Ende des Nutabschnitts mit variablem Radius (412) der vierten Nut (66) gleitet, wenn sich das Drehrad (4) in Richtung des distalen Endes dreht.

8. Vorrichtung zur Einbringung eines Augenimplantats nach Anspruch 1, wobei eine zweite Nut (42) und eine exzentrische erste Pleuelwelle (43) jeweils an den Endflächen auf zwei Seiten des Drehrades (4) vorgesehen sind, eine dritte Nut (55), die der ersten Pleuelwelle (43) entspricht, an einem proximalen Ende der Schubnadel-Pleuelanordnung (5) vorgesehen ist und eine vierte Pleuelwelle (65), die der zweiten Nut (42) entspricht, an einem proximalen Ende der Rückzugs-Pleuelanordnung (6) vorgesehen ist;
die dritte Nut (55) einen Nutabschnitt mit variablem Radius (552) und einen bogenförmigen Nutabschnitt (551) umfasst, und die erste Pleuelwelle (43) von einem Ende des Nutabschnitts mit variablem Radius (552) zu einem Ende des bogenförmigen Nutabschnitts (551) der dritten Nut (55) gleitet, wenn sich das Drehrad (4) in Richtung des distalen Endes dreht; und
die zweite Nut (42) einen bogenförmigen Nutabschnitt (421) und einen Nutabschnitt mit variablem Radius (422) umfasst, und die vierte Pleuelwelle (65) von einem Ende des bogenförmigen Nutabschnitts (421) zu einem Ende des Nutabschnitts mit variablem Radius (422) der zweiten Nut (42) gleitet, wenn sich das Drehrad (4) in Richtung des distalen Endes dreht.

9. Vorrichtung zur Einbringung eines Augenimplantats nach Anspruch 1, wobei eine erste Nut (41) und eine zweite Nut (42) jeweils an zwei Seitenflächen des Drehrades (4) vorgesehen sind, und eine dritte Pleuelwelle (54), die der ersten Nut (41) entspricht, und eine vierte Pleuelwelle (65), die der zweiten Nut (42) entspricht, jeweils an der Schubnadel-Pleuelanordnung (5) und der Rückzugs-Pleuelanordnung (6) vorgesehen sind;
die erste Nut (41) einen Nutabschnitt mit variablem Radius (412) und einen bogenförmigen Nutabschnitt (411) umfasst, und die dritte Pleuelwelle (54) von einem Ende des Nutabschnitts mit variablem Radius (412) zu einem Ende des bogenförmigen Abschnitts (411) der ersten Nut (41) gleitet, wenn sich das Drehrad (4) in distaler Richtung dreht; und
die zweite Nut (42) einen bogenförmigen Nutabschnitt (421) und einen Nutabschnitt mit variablem Radius (422) umfasst, und die vierte Pleuelwelle (65) von einem Ende des bogenförmigen Nutabschnitts (421) zu einem Ende des Nutabschnitts mit variablem Radius (422) der zweiten Nut (42) gleitet, wenn sich das Drehrad (4) in Richtung des distalen Endes dreht.

10. Vorrichtung zur Einbringung eines Augenimplantats nach Anspruch 1, wobei eine exzentrische erste Pleuelwelle (43) und eine exzentrische zweite Pleuelwelle (44) jeweils an zwei Seitenflächen des Drehrades (4) vorgesehen sind;
eine dritte Nut (55), die der ersten Pleuelwelle (43) entspricht, an einem proximalen Abschnitt der Schubnadel-Pleuelanordnung (5) vorgesehen ist und einen Nutabschnitt mit variablem Radius (552) und einen bogenförmigen Nutabschnitt (521) umfasst, und die erste Pleuelwelle (43) von einem Ende des Nutabschnitts mit variablem Radius (552) zu einem Ende des bogenförmigen Nutabschnitts (521) der dritten Nut (55) gleitet, wenn sich das Drehrad (4) in Richtung des distalen Endes dreht; und
eine vierte Nut (66) an einem proximalen Ende der Rückzugs-Pleuelanordnung (6) vorgesehen ist und einen bogenförmigen Nutabschnitt (661) und einen Nutabschnitt mit variablem Radius (662) umfasst, und die zweite Pleuelwelle (44) von einem Ende des bogenförmigen Nutabschnitts (661) zu einem Ende des Nutabschnitts mit variablem Radius (662) der vierten Nut (66) gleitet, wenn sich das Drehrad (4) in Richtung des distalen Endes dreht.

11. Vorrichtung zur Einbringung eines Augenimplantats nach Anspruch 1, wobei das Gehäuse (1) ein oberes Gehäuse und ein fest mit dem oberen Gehäuse verbundenes, unteres Gehäuse umfasst, wobei das obere Gehäuse und das untere Gehäuse jeweils einen Zylinderabschnitt (11), einen Greifabschnitt (12) und einen konischen Abschnitt (13) umfassen, die fest miteinander verbunden sind;
eine Drehradhalterung (17) an einer Innenwandfläche des Zylinderabschnitts (11) des Gehäuses (1) angeordnet ist, und das Drehrad (4) über die Drehwellen (45) an der Drehradhalterung (17) angebracht ist; und
ein Anschlagblock (16) an einer Innenwandfläche mindestens eines des Zylinderabschnitts (11), des Greifabschnitts (12) und des konischen Abschnitts (13) vorgesehen ist, wobei der Anschlagblock (16) so konfiguriert ist, dass er die Schubnadel-Pleuelanordnung (5) und die Rückzugs-Pleuelanordnung (6) in radialer Richtung begrenzt.

12. Vorrichtung zur Einbringung eines Augenimplantats nach Anspruch 1, wobei das Drehrad (4) an einer Außenumfangsfläche mit Zähnen (46) versehen ist und das Gehäuse (1) an einer der Öffnung (15) entsprechenden Stelle mit einer Betätigungs-Gleitbuchse (7) versehen ist, wobei die Betätigungs-Gleitbuchse verschiebbar mit dem Gehäuse (1) verbunden ist; und
die Betätigungs-Gleitbuchse (7) an einer Innenwand mit einem Zahnstangenabschnitt versehen ist, wobei der Zahnstangenabschnitt mit den Zähnen (46) am Drehrad (4) in Eingriff steht.

13. Vorrichtung zur Einbringung eines Augenimplantats nach Anspruch 12, wobei das Gehäuse (1) an einer Außenwand mit einem Federblech (72) versehen ist, wobei sich das Federblech (72) zur Außenseite des Gehäuses (1) erstreckt und mit dem Zahnstangenabschnitt an einer Innenwand der Betätigungs-Gleitbuchse (7) zusammenwirkt.

14. Vorrichtung zur Einbringung eines Augenimplantats nach Anspruch 1, wobei die Schubnadel-Pleuelanordnung (5) und die Rückzugs-Pleuelanordnung (6) jeweils mit einem Positionierungsteil versehen sind, das mit einem Positionierungsloch versehen ist, und die Positionierungslöcher einander gegenüberliegen; und
das Gehäuse (1) mit einem Positionierungsdurchgangsloch an einer den Positionierungslöchern entsprechenden Stelle versehen ist, und in dem Positionierungsdurchgangsloch eine Positionierungsstange vorgesehen ist.

## Revendications

1. Dispositif de délivrance d'implant oculaire, comprenant un boîtier (1), une roue rotative (4), un ensemble de liaison d'aiguille-poussoir (5), un ensemble de liaison de rétraction (6), une gaine (2) et une aiguille à ponction (3),
dans lequel un trou traversant (14) est prévu à une extrémité distale du boîtier (1), et la gaine (2) s'étend à partir d'un intérieur du boîtier (1) vers un extérieur du boîtier (1) à travers le trou traversant (14) et est reliée de manière fixe au boîtier (1) ;
dans lequel l'aiguille à ponction (3) passe à travers la gaine (2), et une extrémité de l'aiguille à ponction (3) s'éloignant d'une pointe de l'aiguille à ponction (3) est reliée de manière fixe à l'ensemble de liaison de rétraction (6) ;
dans lequel une aiguille-poussoir (51) de l'ensemble de liaison d'aiguille-poussoir (5) s'étend à travers un trou interne de l'aiguille à ponction (3), la roue rotative (4) est reliée à un intérieur du boîtier (1) par l'intermédiaire d'arbres rotatifs (45), et une ouverture (15) correspondant à la roue rotative (4) est prévue sur le boîtier (1) ; **caractérisé en ce que**,
l'ensemble de liaison d'aiguille-poussoir (5) et l'ensemble de liaison de rétraction (6) sont reliés de manière mobile à des surfaces d'extrémité de deux côtés de la roue rotative (4) respectivement, de sorte que l'ensemble de liaison d'aiguille-poussoir (5) soit entraîné à se déplacer dans une direction vers l'extrémité distale du boîtier (1) ou que l'ensemble de liaison de rétraction (6) soit entraîné à se déplacer dans une direction s'éloignant de l'extrémité distale du boîtier (1) par la rotation de la roue rotative (4) ;
et dans lequel :
l'ensemble de liaison d'aiguille-poussoir (5) et l'ensemble de liaison de rétraction (6) sont reliés de manière mobile aux surfaces d'extrémité de deux côtés de la roue rotative (4) respectivement par l'intermédiaire d'une structure d'arbre de liaison-rainure, de sorte que, par la rotation de la roue rotative (4), un arbre de liaison de la structure d'arbre de liaison-rainure coulisse le long d'une trajectoire de mouvement définie par une rainure de la structure d'arbre de liaison-rainure pour entraîner l'ensemble de liaison d'aiguille-poussoir (5) à se déplacer dans la direction vers l'extrémité distale du boîtier (1) ou pour entraîner l'ensemble de liaison de rétraction (6) à se déplacer dans une direction s'éloignant de l'extrémité distale du boîtier (1).

2. Dispositif de délivrance d'implant oculaire selon la revendication 1, dans lequel la rainure comprend deux sections de rainure, dont l'une est une section de rainure en arc, et l'autre est une section de rainure à rayon variable ; et
une extrémité de la section de rainure à rayon variable est communiquée avec une extrémité de la section de rainure en arc, et une distance entre un centre de la section de rainure à rayon variable et un centre de la section de rainure en arc se diminue progressivement à partir d'une partie de liaison entre la section de rainure à rayon variable et la section de rainure en arc vers l'autre extrémité de la section de rainure à rayon variable.

3. Dispositif de délivrance d'implant oculaire selon la revendication 1, dans lequel l'ensemble de liaison d'aiguille-poussoir (5) comprend l'aiguille-poussoir (51), un élément de liaison d'aiguille-poussoir (52) et un siège de liaison (53), une extrémité proximale de l'aiguille-poussoir (51) étant reliée de manière fixe à une extrémité distale de l'élément de liaison d'aiguille-poussoir (52), et une extrémité proximale de l'élément de liaison d'aiguille-poussoir (52) étant reliée de manière fixe au siège de liaison (53) ; et
l'un de l'arbre de liaison et de la rainure est prévu sur une surface latérale du siège de liaison (53) opposée à la roue rotative (4), et l'autre de l'arbre de liaison et de la rainure est prévu sur la surface d'extrémité correspondante de la roue rotative (4).

4. Dispositif de délivrance d'implant oculaire selon la revendication 1, dans lequel l'ensemble de liaison de rétraction (6) comprend un connecteur (61), un élément de liaison de rétraction (62) et une partie de liaison (63), le connecteur (61) étant relié de manière fixe à une extrémité distale de l'élément de liaison de rétraction (62), et la partie de liaison (63) étant reliée de manière fixe à une extrémité proximale de l'élément de liaison de rétraction (62) ; et
l'un de l'arbre de liaison et de la rainure est prévu sur une surface latérale de la partie de liaison (63) opposée à la roue rotative (4), et l'autre de l'arbre de liaison et de la rainure est prévu sur la surface d'extrémité correspondante de la roue rotative (4).

5. Dispositif de délivrance d'implant oculaire selon la revendication 1, dans lequel une extrémité proximale de la gaine (2) est reliée de manière fixe à un siège de gaine qui est disposé dans le boîtier (1) à une position proche du trou traversant (14), et la gaine (2) s'étend à travers le trou traversant (14) sur le boîtier (1) vers l'extérieur du boîtier (1).

6. Dispositif de délivrance d'implant oculaire selon la revendication 1, dans lequel un siège d'aiguille à ponction (31) est prévu à une extrémité proximale de l'aiguille à ponction (3) et est relié de manière fixe à une extrémité distale de l'ensemble de liaison de rétraction (6), et l'aiguille à ponction (3) est reliée de manière fixe au siège d'aiguille à ponction (31) et s'étend à travers la gaine (2).

7. Dispositif de délivrance d'implant oculaire selon la revendication 1, dans lequel une première rainure (41) et un deuxième arbre de liaison excentrique (44) sont prévus sur des surfaces d'extrémité de deux côtés de la roue rotative (4) respectivement, un troisième arbre de liaison (54) correspondant à la première rainure (41) est prévu à une extrémité proximale de l'ensemble de liaison d'aiguille-poussoir (5), et une quatrième rainure correspondant au deuxième arbre de liaison (44) est prévue à l'extrémité proximale de l'ensemble de liaison de rétraction (6) ;
la première rainure (41) comprend une section de rainure à rayon variable (412) et une section de rainure en arc (411), et le troisième arbre de liaison (54) coulisse à partir d'une extrémité de la section de rainure à rayon variable (412) vers une extrémité de la section de rainure en arc (411) de la première rainure (41) lors de la rotation de la roue rotative (4) vers l'extrémité distale ; et
la quatrième rainure (66) comprend une section de rainure en arc (411) et une section de rainure à rayon variable (412), et le deuxième arbre de liaison (44) coulisse à partir d'une extrémité de la section de rainure en arc (411) vers une extrémité de la section de rainure à rayon variable (412) de la quatrième rainure (66) lors de la rotation de la roue rotative (4) vers l'extrémité distale.

8. Dispositif de délivrance d'implant oculaire selon la revendication 1, dans lequel une deuxième rainure (42) et un premier arbre de liaison excentrique (43) sont prévus sur des surfaces d'extrémité de deux côtés de la roue rotative (4) respectivement, un troisième rainure (55) correspondant au premier arbre de liaison (43) est prévue à une extrémité proximale de l'ensemble de liaison d'aiguille-poussoir (5), et une quatrième arbre de liaison (65) correspondant au deuxième rainure (42) est prévue à une extrémité proximale de l'ensemble de liaison de rétraction (6) ;
la troisième rainure (55) comprend une section de rainure à rayon variable (552) et une section de rainure en arc (551), et le premier arbre de liaison (43) coulisse à partir d'une extrémité de la section de rainure à rayon variable (552) vers une extrémité de la section de rainure en arc (551) de la troisième rainure (55) lors de la rotation de la roue rotative (4) vers l'extrémité distale ; et
la deuxième rainure (42) comprend une section de rainure en arc (421) et une section de rainure à rayon variable (422), et le quatrième arbre de liaison (65) coulisse à partir d'une extrémité de la section de rainure en arc (421) vers une extrémité de la section de rainure à rayon variable (422) de la deuxième rainure (42) lors de la rotation de la roue rotative (4) vers l'extrémité distale.

9. Dispositif de délivrance d'implant oculaire selon la revendication 1, dans lequel une première rainure (41) et une deuxième rainure (42) sont prévues sur deux surfaces latérales de la roue rotative (4) respectivement, et un troisième arbre de liaison (54) correspondant à la première rainure (41) et un quatrième arbre de liaison (65) correspondant à la deuxième rainure (42) sont prévus sur l'ensemble de liaison d'aiguille-poussoir (5) et l'ensemble de liaison de rétraction (6) respectivement ;
la première rainure (41) comprend une section de rainure à rayon variable (412) et une section de rainure en arc (411), et le troisième arbre de liaison (54) coulisse à partir d'une extrémité de la section de rainure à rayon variable (412) vers une extrémité de la section de rainure en arc (411) de la première rainure (41) lors de la rotation de la roue rotative (4) vers la direction distale ; et
la deuxième rainure (42) comprend une section de rainure en arc (421) et une section de rainure à rayon variable (422), et le quatrième arbre de liaison (65) coulisse à partir d'une extrémité de la section de rainure en arc (421) vers une extrémité de la section de rainure à rayon variable (422) de la deuxième rainure (42) lors de la rotation de la roue rotative (4) vers l'extrémité distale.

10. Dispositif de délivrance d'implant oculaire selon la revendication 1, dans lequel un premier arbre de liaison excentrique (43) et un deuxième arbre de liaison excentrique (44) sont prévus sur deux surfaces latérales de la roue rotative (4) respectivement ;
une troisième rainure (55) correspondant au premier arbre de liaison excentrique (43) est prévue à une partie proximale de l'ensemble de liaison d'aiguille-poussoir (5) et comprend une section de rainure à rayon variable (552) et une section de rainure en arc (521), et le premier arbre de liaison (43) coulisse à partir d'une extrémité de la section de rainure à rayon variable (552) vers une extrémité de la section de rainure en arc (521) de la troisième rainure (55) lors de la rotation de la roue rotative (4) vers l'extrémité distale ; et
une quatrième rainure (66) est prévue à une extrémité proximale de l'ensemble de liaison de rétraction (6) comprend une section de rainure en arc (661) et une section de rainure à rayon variable (662), et le deuxième arbre de liaison (44) coulisse à partir d'une extrémité de la section de rainure en arc (661) vers une extrémité de la section de rainure à rayon variable (662) de la quatrième rainure (66) lors de la rotation de la roue rotative (4) vers l'extrémité distale.

11. Dispositif de délivrance d'implant oculaire selon la revendication 1, dans lequel le boîtier (1) comprend un boîtier supérieur et un boîtier inférieur relié de manière fixe au boîtier supérieur, le boîtier supérieur et le boîtier inférieur comprenant chacun une partie cylindrique (11), une partie de préhension (12) et une partie conique (13) qui sont reliée de manière fixes ensemble ;
un support de montage de roue rotative (17) est disposé sur une surface de paroi interne de la partie cylindrique (11) boîtier (1), et la roue rotative (4) est montée sur le support de montage de roue rotative (17) par l'intermédiaire des arbres rotatifs (45) ; et
un bloc d'arrêt (16) est prévu sur une surface de paroi interne d'au moins l'une parmi la partie cylindrique (11), la partie de préhension (12) et la partie conique (13), et le bloc d'arrêt (16) est configuré pour limiter l'ensemble de liaison d'aiguille-poussoir (5) et le ensemble de liaison de rétraction (6) dans une direction radiale.

12. Dispositif de délivrance d'implant oculaire selon la revendication 1, dans lequel la roue rotative (4) est pourvue de dents (46) sur une surface circonférentielle externe, et le boîtier (1) est pourvu d'une gaine coulissante opérationnelle (7) à une position correspondant à l'ouverture (15), la gaine coulissante opérationnelle étant montée de manière coulissante sur le boîtier (1) ; et
la gaine coulissante opérationnelle (7) est pourvue d'une partie crémaillère sur une paroi interne, la partie crémaillère s'engrenant avec les dents (46) de la roue rotative (4).

13. Dispositif de délivrance d'implant oculaire selon la revendication 12, dans lequel le boîtier (1) est pourvu d'une gaine à ressort (72) sur une paroi externe, la gaine à ressort (72) s'étendant vers l'extérieur du boîtier (1) et coopérant avec la partie crémaillère sur une paroi interne de la gaine coulissante opérationnelle (7).

14. Dispositif de délivrance d'implant oculaire selon la revendication 1, dans lequel l'ensemble de liaison d'aiguille-poussoir (5) et l'ensemble de liaison de rétractation (6) sont chacun pourvus d'une partie de positionnement qui est pourvue d'un trou de positionnement, et les trous de positionnement sont opposés l'un à l'autre ; et
le boîtier (1) est pourvu d'un trou traversant de positionnement à une position correspondant aux trous de positionnement, et une tige de positionnement est prévue dans le trou traversant de positionnement.
